(19) Europäisches Patentamt
European Patent Office
Office européen des brevets

(11) Publication number: **0 039 051**
**B1**

(12) # EUROPEAN PATENT SPECIFICATION

(45) Date of publication of patent specification: **24.07.85**

(21) Application number: **81103066.7**

(22) Date of filing: **23.04.81**

(51) Int. Cl.⁴: **C 07 D 209/28,** C 07 C 83/10, C 07 C 147/14, C 07 D 207/20, C 07 D 207/337, C 07 D 209/26, C 07 D 209/46, C 07 D 211/14, C 07 D 213/82, C 07 D 233/06, C 07 D 233/56

(54) **Mannich-base hydroxamic acid prodrugs for the improved bioavailability of non-steroidal anti-inflammatory agents, a process for preparing and a pharmaceutical composition containing them.**

(30) Priority: **24.04.80 US 143520**

(43) Date of publication of application:
**04.11.81 Bulletin 81/44**

(45) Publication of the grant of the patent:
**24.07.85 Bulletin 85/30**

(84) Designated Contracting States:
**AT BE CH DE FR GB IT LI LU NL SE**

(56) References cited:
**DE-A-2 144 641**
**US-A-3 624 103**
**US-A-4 206 220**

(73) Proprietor: **MERCK & CO. INC.**
**126, East Lincoln Avenue P.O. Box 2000**
**Rahway New Jersey 07065 (US)**

(72) Inventor: **Sloan, Kenneth B.**
**RR 1 Box 71B**
**Eudora Kansas 66025 (US)**
Inventor: **Little, Roy**
**3620 Southwest Archer Road No. CA7**
**Gainesville Florida 32608 (US)**

(74) Representative: **Abitz, Walter, Dr.-Ing. et al**
**Abitz, Morf, Gritschneder P.O. Box 86 01 09**
**D-8000 München 86 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**EP 0 039 051 B1**

Courier Press, Leamington Spa, England.

**Description**

The present invention relates to derivatives of non-steroidal anti-inflammatory drugs characterized as being (1) more readily bioavailable; (2) less irritating to topical and gastric mucosal membranes; and (3) more permeable through topical membranes such as the ophthalmic membrane, skin, when administered orally or topically to warm-blooded animals than are the non-steroidal anti-inflammatory drugs from which they are derived.

For the purposes of this specification, the term "prodrug" denotes a derivative of a known and proven prior art non-steroidal anti-inflammatory compound (e.g., indomethacin, sulindac, naproxen), which derivative, when administered to a warm-blooded animal, "cleaves" in such a manner as to release the proven drug form at its target site or sites of activity. The enzymatic and/or chemical hydrolytic "cleavage" of the compounds of the instant invention occurs in such a manner that the proven drug form (the conventional non-steroidal anti-inflammatory agent) is released while the remaining "cleaved" moiety remains nontoxic and is metabolized in such a manner that nontoxic-metabolic products are produced.

Usually, the un-ionized form of a drug is absorbed more efficiently than its ionic species. In the case of non-steroidal anti-inflammatory drugs containing the carboxylic acid functional group such as indomethacin, sulindac, naproxen, the carboxylic acid is significantly ionized at physiological pH. The result is that such non-steroidal anti-inflammatory drugs are poorly absorbed through lipid-water membrane barriers and are irritating. Thus, an object of the present invention is to provide a class of derivatives of non-steroidal anti-inflammatory drugs which would not be significantly ionized at physiological pH and which are reasonably stable, but which hydrolyze readily *in vivo* and yield upon hydrolysis only the parent non-steroidal anti-inflammatory drug and non-toxic, readily metabolized by-products.

DE—A—2 144 641 relates to phenylacetohydroxamic acids which have anti-inflammatory, analgesic and antipyretic properties. 3-Indoleacetohydroxamic acids which likewise have anti-inflammatory, analgesic and antipyretic activity are known from US—A—3 624 103. It results from the comparative tests set forth further below that the bio-availability of such hydroxamic acids is not satisfactory.

It is one object of the present invention to provide prodrug forms of conventional non-steroidal anti-inflammatory agents which exhibit oral and topical anti-inflammatory activity when administered to warm-blooded animals and are characterized as being more readily bioavailable, less irritating to topical and gastric mucosal membranes and more permeable through topical membranes, e.g., ophthalmic membrane or skin, than are the non-steroidal anti-inflammatory drugs from which they are derived.

It is another object of the present invention to provide such prodrug forms of conventional anti-inflammatory compounds which, following administration, will "cleave" in such a manner as to enable the original parent moiety (indomethacin, sulindac, naproxen, or other known non-steroidal anti-inflammatory agent) to be released at its therapeutic site or sites of anti-inflammatory activity and to further permit the cleaved moiety(ies) unassociated with the parent moiety to be metabolized in a nontoxic fashion.

The foregoing objects are achieved by topically or orally administering to a warm-blooded animal afflicted with inflammation, a therapeutically effective anti-inflammatory amount of a compound having the formula (I):

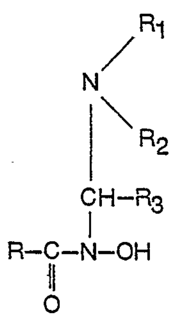

I

wherein

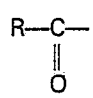

is the acyl residue of any non-steroidal anti-inflammatory agent containing a carboxylic acid function; $R_1$ and $R_2$, which can be the same or different, each represent a member selected from the group consisting of alkyl of 1 to 20 carbon atoms; aryl of 6 to 10 carbon atoms; cycloalkyl of 3 to 8 carbon atoms; aralkyl, alkaryl, alkenylaryl, and alkynylaryl, wherein the alkyl, and aryl portions are defined as above and the alkenyl and alkynyl portions have 2—20 carbon atoms; and substituted derivatives of the above-defined alkyl, aryl, cycloalkyl, aralkyl, alkaryl, alkenylaryl and alkynylaryl radicals, said derivatives having one or

more substituents each of which are selected from the group consisting of $C_1$—$C_8$ alkyl, $C_1$—$C_8$ alkanoyl, cyano, $C_2$—$C_9$ carbalkoxy, $C_1$—$C_8$ haloalkyl having 1 or more halo substituents, carboxyl, dialkylcarbamyl wherein the alkyl portions each contain 1 to 8 carbon atoms, and $C_1$—$C_8$ alkylsulfonyl; or $R_1$ or $R_2$ are combined so that —$NR_1R_2$ represents the residue of a saturated or unsaturated heterocyclic compound; $R_3$ is selected from the group consisting of hydrogen, $R_1$,

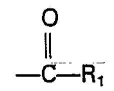

—$CH_2OCOR_1$, —$CH_2ONO_2$, pyridyl, furyl, cyano, carbamyl, $C_2$—$C_9$ alkylcarbamyl and dialkylcarbamyl wherein the alkyl portions each contain 1 to 8 carbon atoms; $R_1$ is any radical encompassed by the definition of $R_1$ above; or a non-toxic pharmaceutically acceptable acid addition salt or a non-toxic N-oxide thereof and/or sulfoxides in case —$NR_1R_2$ represents a sulfur containing heterocycle.

The term "nontoxic pharmaceutically acceptable acid addition salt" as used herein generally includes the nontoxic acid addition salts of selected compounds of formula (I), formed with nontoxic inorganic or organic acids. For example, the salts include those derived from inorganic acids such as hydrochloric, hydrobromic, sulfuric, sulfamic, phosphoric, nitric acid; and the salts prepared from organic acids such as acetic, propionic, succinic, glycollic, stearic, lactic, malic, tartaric, citric, ascorbic, pamoic, maleic, hydroxymaleic, phenylacetic, glutamic, benzoic, salicylic, sulfanilic, fumaric, methanesulfonic, toluenesulfonic acid.

Usually, the oxides are N-oxides but they may be sulfoxides in the event that —$NR_1R_2$ represents the residue of a sulfur-containing heterocycle. The oxides are prepared by oxidation of the selected compounds of formula (I) with a suitable oxidizing agent, e.g., *m*-chloroperbenzoic acid or hydrogen peroxide, in an appropriate inert solvent such as dichloromethane, benzene or chloroform.

The term "acyl residue" as used herein with respect to any non-steroidal anti-inflammatory agent containing a carboxylic acid function is intended to represent that portion of the anti-inflammatory compound which remains after removal of the —OH from the —COOH portion of the molecule.

The chemical structure of the non-steroidal anti-inflammatory drugs whose acyl residues are encompassed by formula (I) is not critical, so long as those drugs contain a carboxylic acid function. Suitable anti-inflammatory agents from which the instant prodrugs are derived include, but are not limited to, indomethacin, aspirin, naproxen, fenoprofen, sulindac, ibuprofen, tolmetin, difunisal, flurbiprofen, indoprofen, mefanamic acid, fenclozic acid, ketoprofen, alcolfenac, bucloxic acid, meclofenamic acid, flufenamic acid, cinchophen, voltaren, cinmetacin, ibufenac, furobufen, fenclofenac, prodolic acid, pirprofen, oxoprozin, clonixin, fluprofen and flutiazin.

With respect to the radicals encompassed by $R_1$, $R_2$ and —$NR_1R_2$ in formula (I) and throughout this specification, the following definitions are applicable:

The expression "the residue of a saturated or unsaturated heterocyclic compound" as used herein is intended to indicate the portion of the heterocyclic compound which remains after removal of the hydrogen atom from the secondary nitrogen. Thus, the term "saturated or unsaturated heterocyclic compound" encompasses saturated monocycles containing one or more hetero atoms in the ring, optionally bearing one or more substituents such as phenyl, benzyl and methyl; and unsaturated one and two ring systems containing one or more double bonds and one or more ring hetero atoms, optionally substituted with one or more methyl groups. In every case, the substituents and double bonds, if any, must be located such that the parent heterocyclic compound from which the residue —$NR_1R_2$ is derived is a compound of the formula $HNR_1R_2$, i.e., the parent compound invariably contains one N which is a secondary nitrogen atom. When the parent heterocycle contains more than one nitrogen atom, one nitrogen must be a secondary nitrogen and any other nitrogen must be tertiary. In any case, the parent compound can contain other hetero atoms, e.g., sulfur and/or oxygen. Illustrative of residues of saturated monocyclic heterocyclics which are encompassed by the —$NR_1R_2$ term are morpholino, perhydro-1,2,4-oxathiazin-4-yl, 1-pyrrolidinyl, 4-benzyl-1-piperazinyl, 4-methyl-1-piperazinyl, piperidino, hexamethyleneimino, 4-phenylpiperidino, 2-methyl-1-pyrazolidinyl, 3-methyl-1-imidazolidinyl, 4-benzylpiperidino and 4-phenyl-1-piperazinyl. Exemplary of residues of unsaturated one and two ring heterocyclic systems represented by —$NR_1R_2$ are radicals such as 1-imidazolyl, 1-pyrrolyl, 1-pyrazolyl, 1-indolinyl, 2-isoindolinyl, 1-indolyl, 2-isoindolyl, 1H-indazol-1-yl, and 7-purinyl; and radicals derived from compounds such as pyrazoline, pyrroline and imidazoline wherein there is one secondary nitrogen atom, e.g., radicals of the type

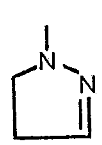 , 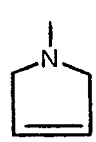 , 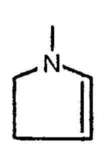 , 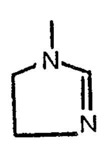

and the like. In addition, any of the aforesaid unsaturated one and two ring heterocycles can be optionally substituted with one or more methyl groups.

The term "aryl" as used herein can be exemplified by phenyl and naphthyl.

In addition, here and throughout this specification, the following examples are applicable: The alkyl radicals include methyl, ethyl, propyl, butyl, pentyl, hexyl, heptyl and octyl and the branched-chain isomers thereof, as well as their straight and branched-chain higher homologues in the instances where "alkyl" can contain more than 8 carbon atoms. The alkenyl and alkynyl portions in the alkenyl aryl and alkynyl aryl substituents may be straight or branched-chain, for example, vinyl, propenyl, butenyl, ethynyl, propynyl, butynyl. The cycloalkyl radicals are exemplified by cyclopentyl and cyclohexyl. The aralkyl radicals are of the type -alkylene-aryl wherein aryl is as defined above and the alkylene moieties contain up to 20 carbon atoms (preferably up to 6 carbon atoms) and can be straight or branched-chain. The alkylene moieties are typified by methylene, ethylene, propylene, trimethylene, 1,2-butylene, 2,3-butylene, tetramethylene and the like. Additionally, the alkanoyl, carbalkoxy, dialkylcarbamyl, alkylsulfonyl and alkylcarbamyl radicals are of the type

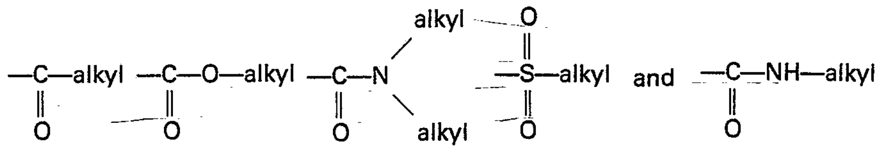

respectively, wherein the alkyl group in each instance contains 1 to 8 carbon atoms.

While all of the compounds encompassed by formule (I) above essentially satisfy the objectives of the present invention, the following selected compounds are preferred:

1. α-[1-(4'-Chlorobenzoyl)-5-methoxy-2-methylindol-3-yl]-N-(morpholinomethyl)acetohydroxamic acid.
2. α-[1-(4'-Chlorobenzoyl)-5-methoxy-2-methylindol-3-yl]-N-(N',N'-diethylaminomethyl)-acetohydroxamic acid.
3. α-[1-(4'-Chlorobenzoyl)-5-methoxy-2-methylindol-3-yl]-N-[(4-methylpiperazin-1-yl)methyl]-acetohydroxamic acid.
4. α-[1-(4'-Chlorobenzoyl)-5-methoxy-2-methylindol-3-yl]-N-(piperidinomethyl)acetohydroxamic acid.
5. α-[1-(4'-Chlorobenzoyl)-5-methoxy-2-methylindol-3-yl]-N-[(pyrrolidin-1-yl)methyl]acetohydroxamic acid.
6. α-[1-(4'-Chlorobenzoyl)-5-methoxy-2-methylindol-3-yl]-N-(N',N'-dicyclohexylaminomethyl)-acetohydroxamic acid.
7. α-[1-(4'-Chlorobenzoyl)-5-methoxy-2-methylindol-3-yl]-N-[(pyrrol-1-yl)methyl]acetohydroxamic acid.
8. α-[1-(4'-Chlorobenzoyl)-5-methoxy-2-methylindol-3-yl]-N-[(indol-1-yl)methyl]acetohydroxamic acid.
9. α-[1-(4'-Chlorobenzoyl)-5-methoxy-2-methylindol-3-yl]-N-[(isoindol-2-yl)methyl]acetohydroxamic acid.
10. α-[1-(4'-Chlorobenzoyl)-5-methoxy-2-methylindol-3-yl]-N-[(purin-7-yl)methyl]acetohydroxamic acid.
11. α-[1-(4'-Chlorobenzoyl)-5-methoxy-2-methylindol-3-yl]-N-(N',N'-dimethylaminomethyl)-acetohydroxamic acid.
12. α-[1-(4'-Chlorobenzoyl)-5-methoxy-2-methylindol-3-yl]-N-(N',N'-diisopropylaminomethyl)-acetohydroxamic acid.
13. α-[1-(4'-Chlorobenzoyl)-5-methoxy-2-methylindol-3-yl]-N-(N',N'-dipentylaminomethyl)-acetohydroxamic acid.
14. α-[1-(4'-Chlorobenzoyl)-5-methoxy-2-methylindol-3-yl]-N-(N',N'-dioctylaminomethyl)-acetohydroxamic acid.
15. α-[*cis*-5-Fluoro-2-methyl-1-[4'-(methylsulfinyl)-benzylidene]inden-3-yl]-N-(piperidinomethyl)-acetohydroxamic acid.
16. α-[*cis*-5-Fluoro-2-methyl-1-[4'-(methylsulfinyl)benzylidene]inden-3-yl]-N-[(N'-benzyl-N'-phenyl)aminomethyl]acetohydroxamic acid.
17. α-[*cis*-5-Fluoro-2-methyl-1-[4'-(methylsulfinyl)-benzylidene]inden-3-yl]-N-(morpholinomethyl)-acetohydroxamic acid.
18. α-[*cis*-5-Fluoro-2-methyl-1-[4'-(methylsulfinyl)benzylidene]inden-3-yl]-N-[(imidazol-1-yl)methyl]-acetohydroxamic acid.
19. α-(6-Methoxynaphthalen-2-yl)-α-methyl-N-(N',N'-diethylaminomethyl)acetohydroxamic acid.
20. α-Methyl-α-(3-phenoxy)phenyl-N-(piperidinomethyl)acetohydroxamic acid.
21. α-Methyl-α-(3-phenoxy)phenyl-N-[(N'-benzyl-N'-phenyl)aminomethyl]acetohydroxamic acid.
22. α-Methyl-α-(3-phenoxy)phenyl-N-(morpholinomethyl)acetohydroxamic acid.
23. α-Methyl-α-(3-phenoxy)phenyl-N-[(imidazol-1-yl)methyl]acetohydroxamic acid.
24. α-Methyl-α-[4-(2'-methylpropyl)]-phenyl-N-(morpholinomethyl)acetohydroxamic acid.
25. α-Methyl-α-[4-(2'-methylpropyl)]-phenyl-N-(piperidinomethyl)acetohydroxamic acid.
26. α-[1-Methyl-5-(p-toluoyl)pyrrol-2-yl]-N-(N',N'-diethylaminomethyl)acetohydroxamic acid.
27. α-Methyl-α-[4-(1'-oxoisoindol-2'-yl)]-phenyl-N-(piperidinomethyl)acetohydroxamic acid.

28. α-Methyl-α-[4-(1′-oxoisoindol-2′-yl)]phenyl-N-(morpholinomethyl)acetohydroxamic acid.

It will be apparent to those skilled in the art that the preferred compounds listed above are derived from indomethacin, sulindac, naproxen, fenoprofen, ibuprofen, tolmetin and indoprofen. Other suitable anti-inflammatory agents from which the instant prodrugs can be derived include, fenclozic acid, ketoprofen, alcolfenac, bucloxic acid, meclofenamic acid, flufenamic acid, cinchophen, voltaren, cinmetacin, ibufenac, furobufen, fenclofenac, prodolic acid, pirpofen, oxoprozin, clonixin, fluprofen, flutiazin, aspirin, diflunisal, flurbiprofen and mefenamic acid.

The novel prodrugs of formula (I) can be prepared by first reacting the acid chloride of a non-steroidal anti-inflammatory agent of the formula II

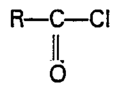 II

wherein

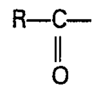

is as hereinbefore defined, with hydroxylamine, suitably employed in the form of its hydrochloride $H_2NOH \cdot HCl$. The reaction is conveniently conducted in the presence of a suitable base, e.g., potassium carbonate, in an appropriate organic solvent or mixture of solvents, e.g., diethyl ether/tetrahydrofuran. The resultant intermediate is of the formula III

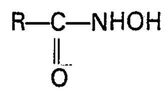 III

wherein

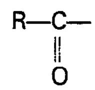

is as hereinbefore defined. The hydroxamic acid of formula (III) is then reacted with formaldehyde or another aldehyde of the type $R_3CHO$ wherein $R_3$ is as defined above, and a secondary amine of the formula IV

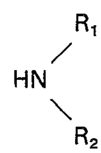 IV

wherein $R_1$, $R_2$ and $—NR_1R_2$ are defined as before, to afford the desired prodrug of formula (I). The reaction is carried out in an appropriate organic solvent such as tetrahydrofuran; formaldehyde can be conveniently introduced into the reaction mixture in the form of formalin (a solution of about 37% by weight of formaldehyde gas in water, usually with 10—15% methanol added to prevent polymerisation).

The acid chloride starting material of formula II above is prepared in known manner from the corresponding nonsteroidal anti-inflammatory agent

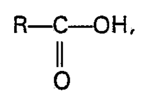

wherein

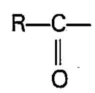

is defined as above, typically by treatment of that acid with thionyl chloride.

The following examples illustrate the invention.

Example 1 (starting material)

Preparation of the Acid Chloride of 1-(4'-Chlorobenzoyl)-5-methoxy-2-methyl-3-indolylacetic Acid, i.e., Indomethacin Acid Chloride

To 9.00 g (0.0757 mol) of thionyl chloride in 400 ml of dichloromethane were added 5.55 g (0.076 mol) of dimethylformamide in 100 ml of dichloromethane. After 10 minutes at room temperature, the above solution was allowed to react with a dichloromethane (100 ml) suspension containing 25.7 g (0.072 mol) of indomethacin. The resulting faintly orange solution was immediately concentrated *in vacuo* to give a light yellow residue which was triturated with 500 ml of ether overnight. The precipitate was then removed by filtration and dried to give 16.7 g (mp 124—126°C, 61% yield) of the desired indomethacin acid chloride: IR (KBr) 1790 and 1675 $cm^{-1}$ (s) (C=O); NMR ($CDCl_3$) δ 7.60 (AB quartet, 4, *J*=9 Hz, $\Delta_{AB}$=11 Hz, aromatic *H*), 7.0—6.55 (m, 3, aromatic H), 4.17 (s, 2, $\underline{C}H_2COCl$) 3.83 (s, 3, O—$C\underline{H}_3$) and 2.41 (s, 3, $C\underline{H}_3$—C=C).

| | | | |
|---|---|---|---|
| *Anal.* Calcd for $C_{19}H_{15}Cl_2NO_3$: | C, 60.65; | H, 4.02; | N, 3.72. |
| Found: | C, 60.59; | H, 4.08; | N, 3.50. |

Example 2

Preparation of α-[1-(4'-Chlorobenzoyl)-5-methoxy-2-methylindol-3-yl]acetohydroxamic Acid, i.e, Indomethacin Hydroxamic Acid

Hydroxylamine hydrochloride (7.47 g, 0.107 mole) and potassium carbonate (14.84 g, 0.107 mole) were dissolved in 20 ml of $H_2O$. 1000 ml of ethyl ether were added and the resulting suspension was stirred for 10 minutes. Then, a suspension of indomethacin acid chloride (40.46 g, 0.107 mole) in 600 ml of tetrahydrofuran was added and the reaction mixture was stirred overnight. The suspension was then filtered and the residue was washed twice with ethyl ether, then was suspended in 600 ml of boiling tetrahydrofuran and filtered while hot. The filtrate was concentrated to 150 ml and allowed to crystallize at room temperature overnight. The crystals were removed by filtration and washed twice with ethyl ether to give 18.72 g (mp 147—150°C, 47% yield) of slightly yellow fibrous crystals which were shown to be the pure product: NMR (DMSO-$d_6$) δ 10.65 (s, 1, NO$\underline{H}$), 8.8 (bm, 1, —N$\underline{H}$OH), 7.67 (s, 4, —$C_6\underline{H}_4Cl$), 7.22—6.6 (m 3, —$C_6\underline{H}_3OCH_3$), 3.80 (s, 3, —$OC\underline{H}_3$), 3.42 (s, 2, O=$CC\underline{H}_2$—), 2.28 (s, 3, $CC\underline{H}_3$), IR (KBr) 3300—3150 $cm^{-1}$ (b) (NO—H), 1650, 1630 $cm^{-1}$ (N—C=O).

| | | | |
|---|---|---|---|
| *Anal.* Calcd for $C_{19}H_{17}ClN_2O_4$: | C, 61.21; | H, 4.60; | N, 7.52. |
| Found: | C, 61.32; | H, 4.80; | N, 7.00. |

Similarly prepared from their acid chlorides are the hydroxamic acid derivatives of sulindac, naproxen, fenoprofen, ibuprofen, tolmetin and indoprofen. The corresponding hydroxamic acid derivatives of the other anti-inflammatory agents specified herein can be prepared in like manner.

Example 3

Preparation of α-[1-(4'-Chlorobenzoyl)-5-methoxy-2-methylindol-3-yl]-N-(morpholinomethyl)aceto hydroxamic Acid

Formalin (0.82 g) was mixed with morpholine (0.71 g, 0.0082 mole) in 90 ml of tetrahydrofuran, then indomethacin hydroxamic acid (3.04 g, 0.0082 mole) was added. The resulting suspension was heated in a hot water bath until all the solids had dissolved, then the reaction vessel was stoppered and the solution was allowed to cool overnight. During that time, a small amount of crystals formed. The solution was diluted to 250 ml with cyclohexane and allowed to stand for 2 hours. The crystals which formed were removed by filtration and washed twice with cyclohexane, then dried *in vacuo* (60°C for 8 hours) to give 2.01 g (mp 179—180°C, 68% yield) of white powder which was pure product: NMR (DMSO-$d_6$) δ 7.70 (s, 4, —$C_6\underline{H}_4Cl$), 7.10—6.70 (m, 3, —$OC_6\underline{H}_3$), 4.35 (s, 2, $NC\underline{H}_2N$), 3.84 (s, 2, —$COC\underline{H}_2$—), 3.77 (s, 3, —$OC\underline{H}_3$), 3.26 (m, 4, O($C\underline{H}_2$)$_2$—), 2.6 (m, 4, N($C\underline{H}_2$)$_2$—), 2.24 (s, 3, C—$C\underline{H}_3$); IR (KBr), 3150 $cm^{-1}$ (bw) (—NO—H), 2900 $cm^{-1}$ (C—H), 1660 $cm^{-1}$ (s) (NC=O), 1600 $cm^{-1}$ (s) (HONC=O).

| | | | |
|---|---|---|---|
| *Anal.* Calcd for $C_{24}H_{25}ClN_3O_5$: | C, 61.21; | H, 5.35; | N, 8.92. |
| Found: | C, 61.44; | H, 5.68; | N, 8.50. |

Example 4

Preparation of α-[1-(4'-Chlorobenzoyl)-5-methoxy-2-methylindol-3-yl]-N-[(4-methylpiperazin-1-yl)methyl] acetohydroxamic Acid

This preparation was carried out analogously to the procedure of Example 3 above, using 3.31 g (0.0089 mole) of indomethacin hydroxamic acid, 0.89 g of formalin and 0.89 g (0.0089 mole) of N-methylpiperazine in 90 ml of tetrahydrofuran. Crystallization of the product was accomplished by concentrating the reaction solution to 30 ml and then diluting that solution to 100 ml with cyclohexane. The resultant cloudy solution was allowed to crystallize over 3 days' time, then the crystals were removed by filtration and washed twice with a mixture of hexanes to give 3.53 g (82% yield, mp 143—145°C) of off-white, powdery crystalline product: NMR (DMSO-$d_6$) δ 7.66 (s, 4, —$C_6\underline{H}_4Cl$), 7.10—6.60 (m, 3, —$OC_6\underline{H}_3$—), 4.33 (s,

2, —$NC\underline{H}_2N$), 3.48 (s, 2, $COC\underline{H}_2$—), 3.41 (s, 3, —$OC\underline{H}_3$), 2.7—2.0 (m, 8, —$N(C\underline{H}_2C\underline{H}_2)_2N$—), 2.19 (s, 3, $NC\underline{H}_3$), 2.16 (s, 3, C—$C\underline{H}_3$); IR (KBrp, 2800—3000 $cm^{-1}$ (m) (C—H), 1665 $cm^{-1}$ (s) (NC=O), 1600 $cm^{-1}$ (s) (HONC=O).

| | | | |
|---|---|---|---|
| *Anal.* Calcd for $C_{25}H_{29}ClN_4O_4$: | C, 61.91; | H, 6.03; | N, 11.56. |
| Found: | C, 62.07; | H, 6.00; | N, 11.21. |

Example 5

Preparation of α-[1-(4′-Chlorobenzoyl)-5-methoxy-2-methylindol-3-yl]-N-(N′,N′-diethylaminomethyl)aceto hydroxamic Acid

This preparation and crystallization were carried out analogously to the procedures of Examples 3 and 4 above, using 3.15 g (0.0085 mole) of indomethacin hydroxamic acid, 0.85 g of formalin and 0.62 (0.0085 mole) of diethylamine in 70 ml of tetrahydrofuran. After 3 days of crystallizing, the crystalline product was removed by filtration and washed twice with a mixture of hexanes to give 3.08 g (79% yield, mp 134—137°C) of white powder: NMR ($CDCl_3$) δ 8.03 (bs, 1, $NO\underline{H}$), 7.7—6.7 (m, 7, aromatic-*H*, 4.42 (s, 2, $NC\underline{H}_2N$), 3.85 (s, 2, —$COC\underline{H}_2$), 3.82 (s, 3, —$OC\underline{H}_3$), 2.65 (q, 4, J=8 Hz, $NC\underline{H}_2$), 2.35 (s, 3, C—$C\underline{H}_3$), 1.04 (t, 6, J=8 Hz, $NCH_2C\underline{H}_3$); IR (KBr) 3150 $cm^{-1}$ (w) (NO—H), 2800—3000 $cm^{-1}$ (w) (C—H), 1667 $cm^{-1}$ (s) (NC=O), 1600 $cm^{-1}$ (s) (HONC=O).

| | | | |
|---|---|---|---|
| *Anal.* Calcd for $C_{24}H_{28}ClN_3O_4$: | C, 62.94; | H, 6.16; | N, 9.18. |
| Found: | C, 62.51; | H, 6.18; | N, 8.90. |

In similar fashion, the other compounds of the present invention can be prepared with similar success by merely following the preceding examples and substituting the appropriate generically and/or specifically described reactants and/or operating conditions of this invention for those of the preceding examples. Thus, the following additional compounds can be prepared by following the above reaction scheme:

COMPOUNDS OF FORMULA (I)

| Example Number | 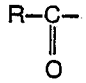 | $R_3$ | 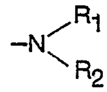 |
|---|---|---|---|
| 6 | 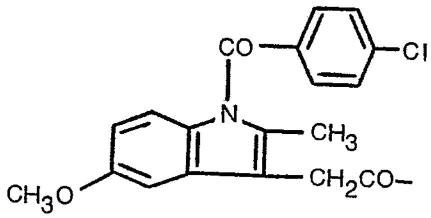 | H | 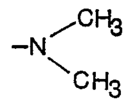 |
| 7 | 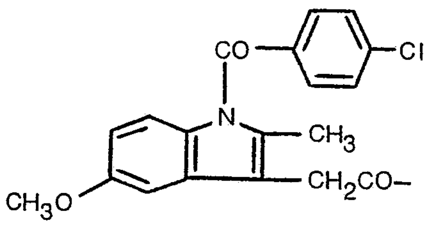 | H | 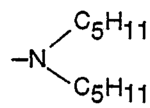 |
| 8 | 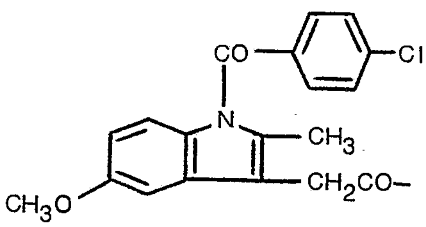 | H | 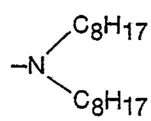 |
| 9 | 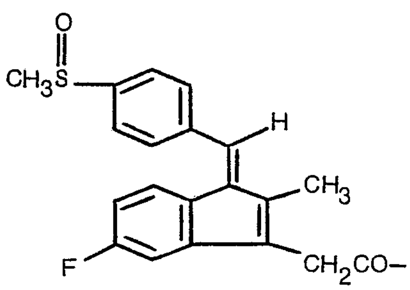 | H | 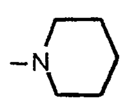 |
| 10 | 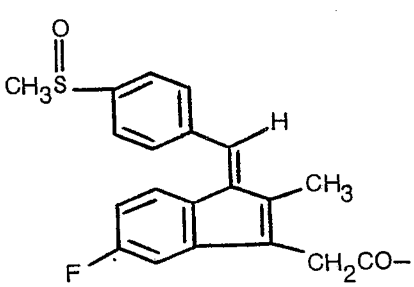 | H | 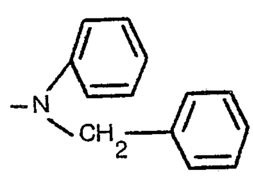 |

COMPOUNDS OF FORMULA (I) (Continued)

| Example Number | 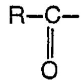 | R3 | 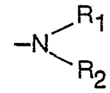 |
|---|---|---|---|
| 11 | 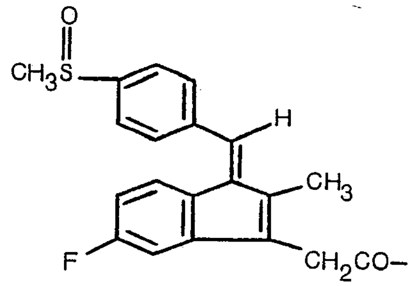 | H | 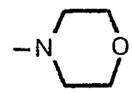 |
| 12 | 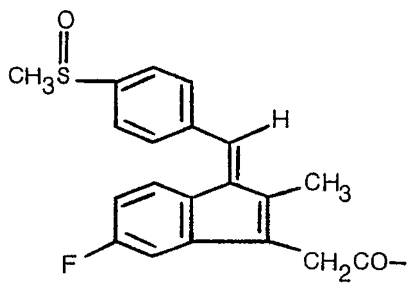 | H | 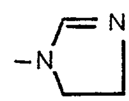 |
| 13 | 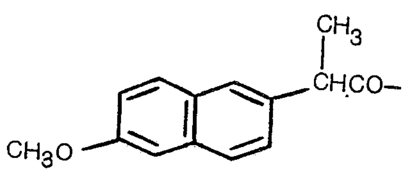 | H | 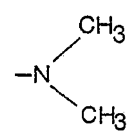 |
| 14 | 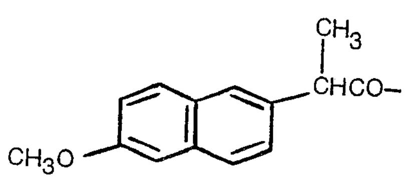 | H | 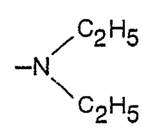 |

COMPOUNDS OF FORMULA (I) (Continued)

| Example Number | 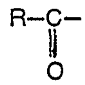 | $R_3$ | 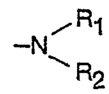 |
|---|---|---|---|
| 15 | 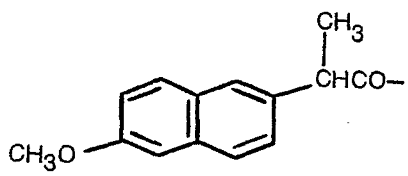 | H | 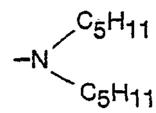 |
| 16 | 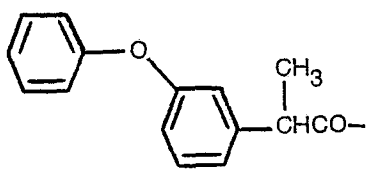 | H | 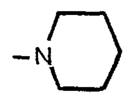 |
| 17 | 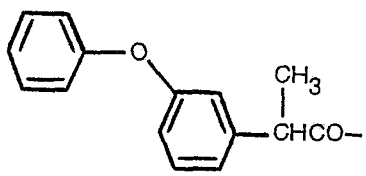 | H | 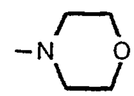 |
| 18 | 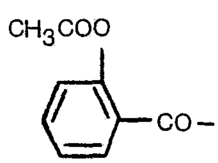 | H | 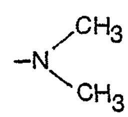 |
| 19 | 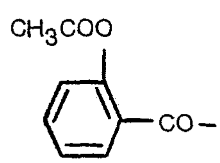 | H | 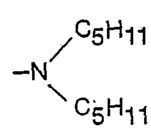 |

COMPOUNDS OF FORMULA (I) (Continued)

| Example Number | 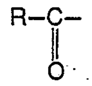 | $R_3$ | 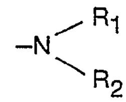 |
|---|---|---|---|
| 20 | 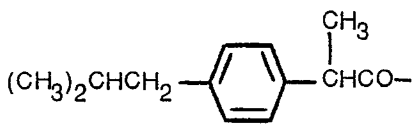 | H | 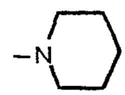 |
| 21 | 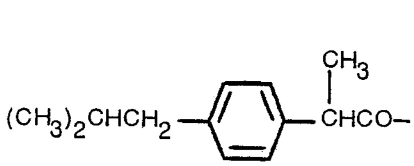 | H | 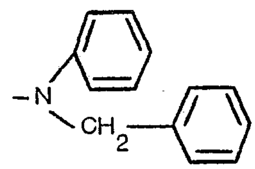 |
| 22 | 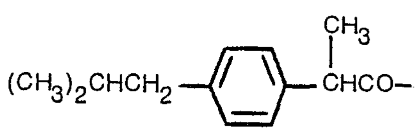 | H | 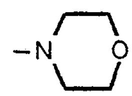 |
| 23 | 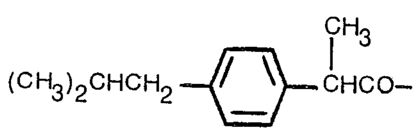 | H | 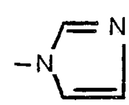 |
| 24 | 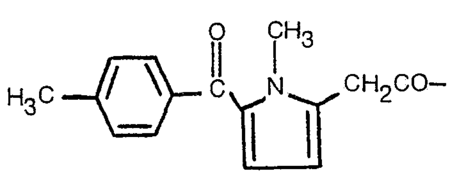 | H | 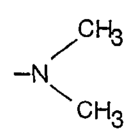 |

COMPOUNDS OF FORMULA (I) (Continued)

| Example Number | 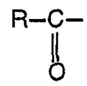 | $R_3$ | 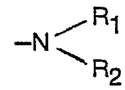 |
|---|---|---|---|
| 25 | 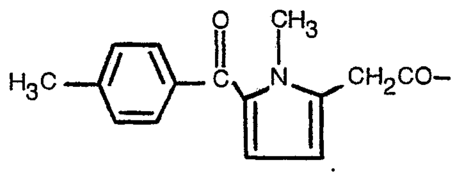 | H | 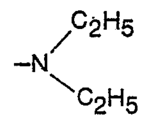 |
| 26 | 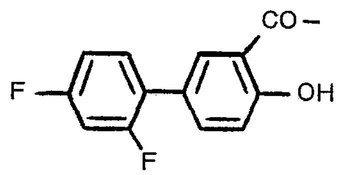 | H | 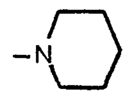 |
| 27 | 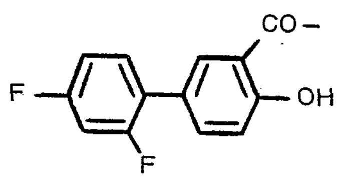 | H | 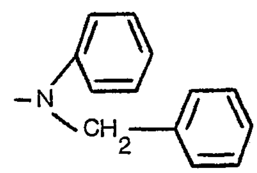 |
| 28 | 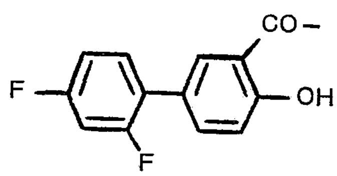 | H | 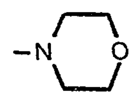 |
| 29 | 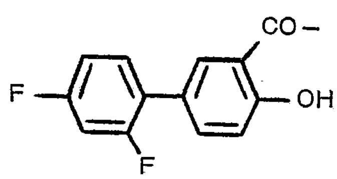 | H | 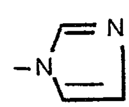 |
| 30 | 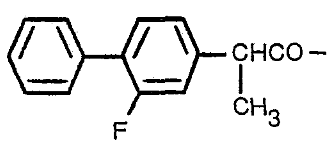 | H | 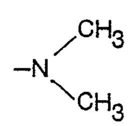 |

COMPOUNDS OF FORMULA (I) (Continued)

| Example Number | 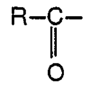 | $R_3$ | 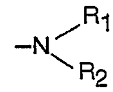 |
|---|---|---|---|
| 31 | 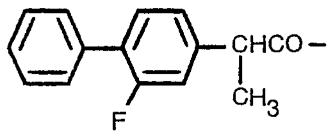 | H | 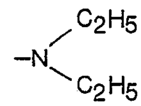 |
| 32 | 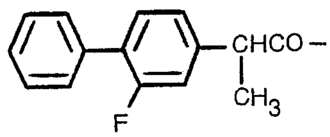 | H | 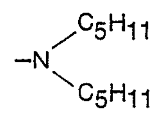 |
| 33 | 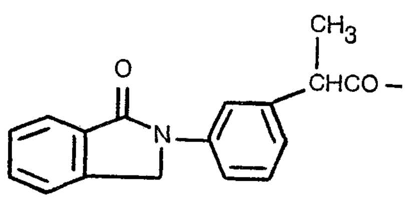 | H | 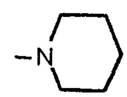 |
| 34 | 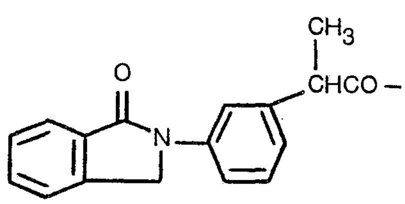 | H | 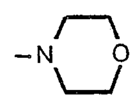 |
| 35 | 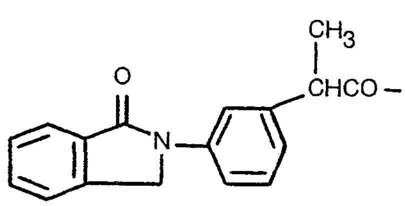 | H | 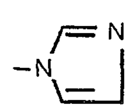 |

COMPOUNDS OF FORMULA (I) (Continued)

| Example Number | 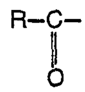 | $R_3$ | 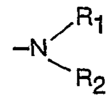 |
|---|---|---|---|
| 36 | 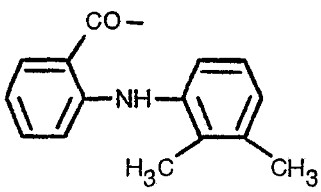 | H | 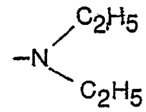 |
| 37 | 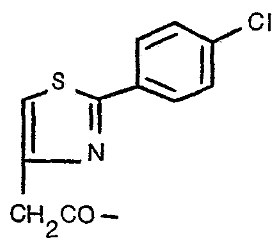 | H | 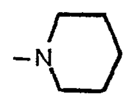 |
| 38 | 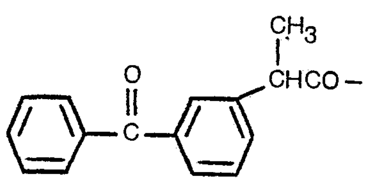 | H | 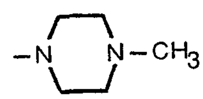 |
| 39 | 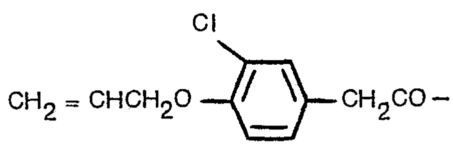 | H | 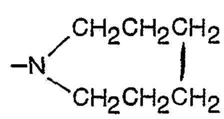 |
| 40 | 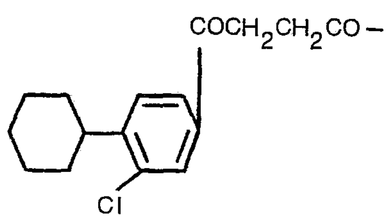 | H | 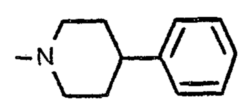 |

COMPOUNDS OF FORMULA (I) (Continued)

| Example Number | 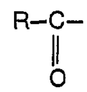 | $R_3$ | 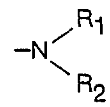 |
|---|---|---|---|
| 41 | 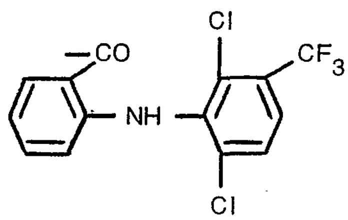 | H | 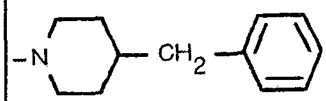 |
| 42 | 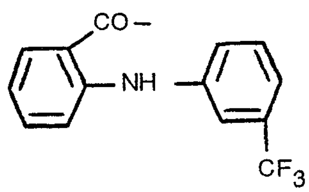 | H | 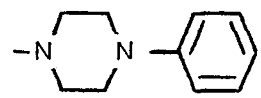 |
| 43 | 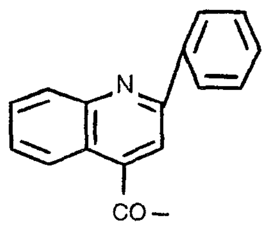 | H | 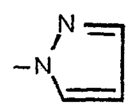 |
| 44 | 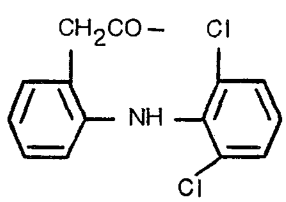 | H | 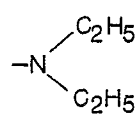 |
| 45 | 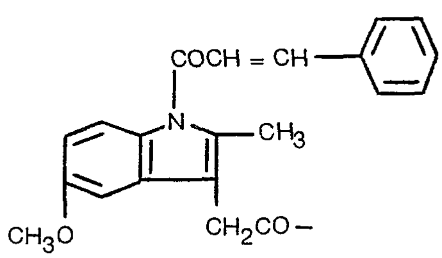 | H | 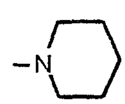 |

COMPOUNDS OF FORMULA (I) (Continued)

| Example Number | 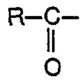 | $R_3$ | 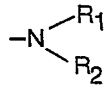 |
|---|---|---|---|
| 46 | 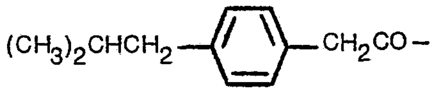 | H | 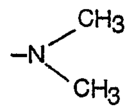 |
| 47 | 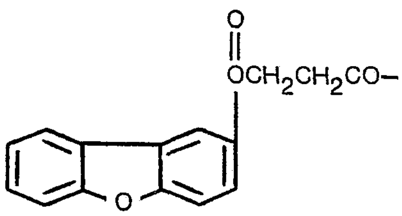 | H | 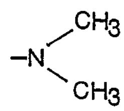 |
| 48 | 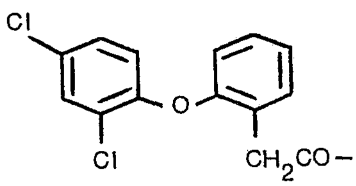 | H | 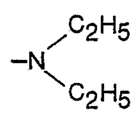 |
| 49 | 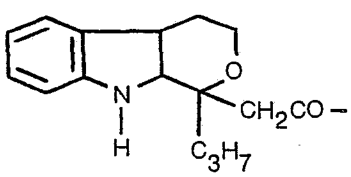 | H | 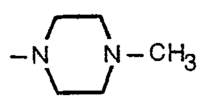 |
| 50 | 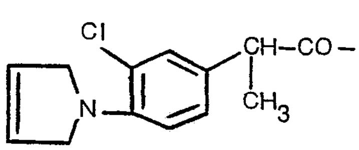 | H | 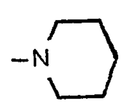 |

COMPOUNDS OF FORMULA (I) (Continued)

| Example Number | 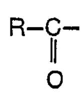 | $R_3$ | 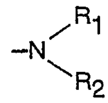 |
|---|---|---|---|
| 51 | 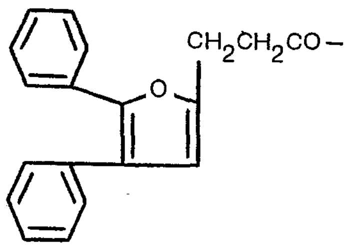 | H | 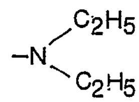 |
| 52 | 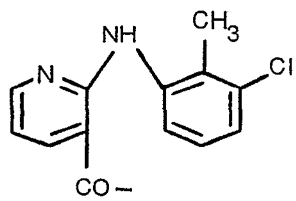 | H | 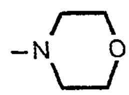 |
| 53 | 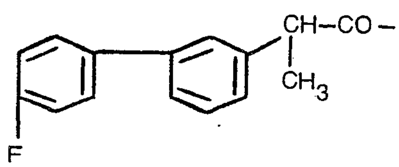 | H | 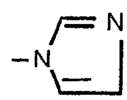 |
| 54 | 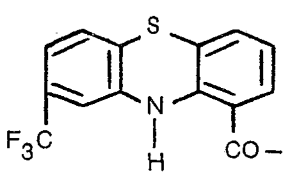 | H | 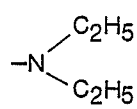 |
| 55 | 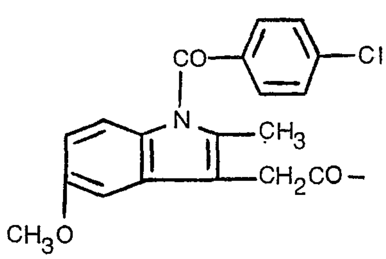 | H | 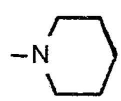 |

COMPOUNDS OF FORMULA (I) (Continued)

| Example Number | 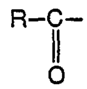 | $R_3$ | 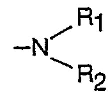 |
|---|---|---|---|
| 56 | 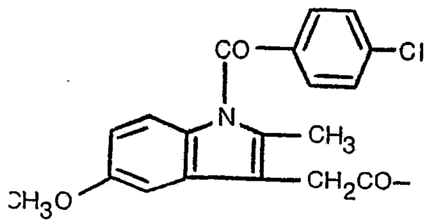 | H | 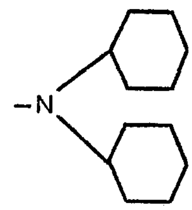 |
| 57 | 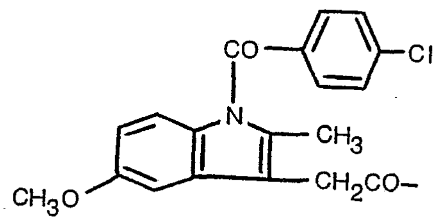 | H | 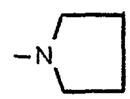 |
| 58 | 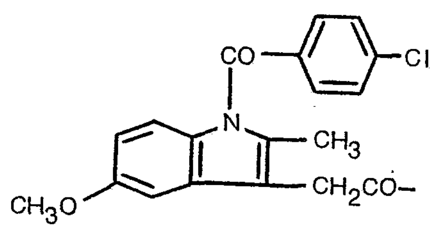 | H | 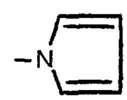 |
| 59 | 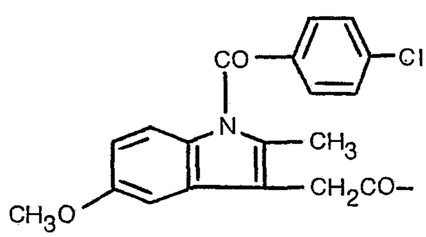 | H | 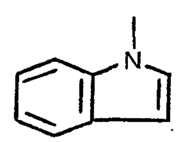 |
| 60 | 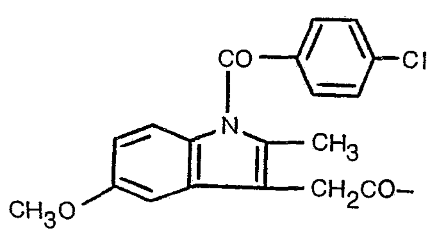 | H | 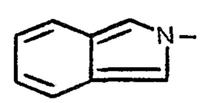 |

COMPOUNDS OF FORMULA (I) (Continued)

| Example Number | 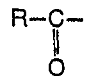 | $R_3$ | 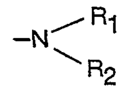 |
|---|---|---|---|
| 61 | 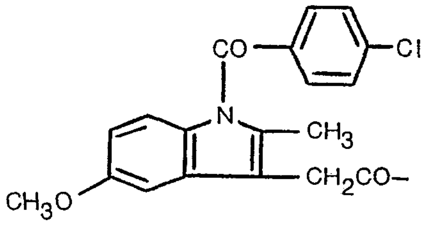 | H | 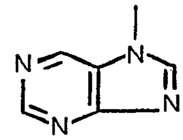 |
| 62 | 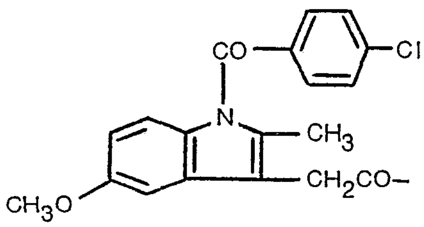 | H | 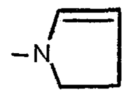 |
| 63 | 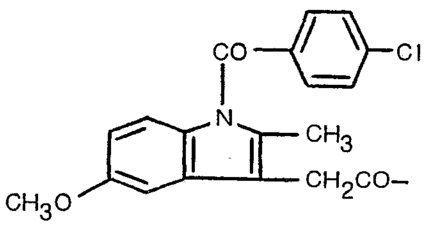 | H | 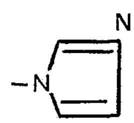 |
| 64 | 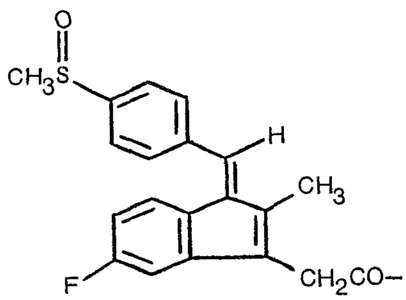 | H | 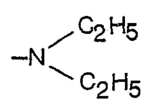 |
| 65 | 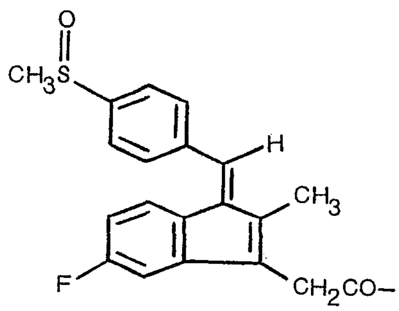 | H | 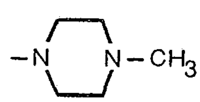 |

COMPOUNDS OF FORMULA (I) (Continued)

| Example Number | 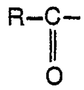 | $R_3$ | 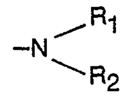 |
|---|---|---|---|
| 66 | 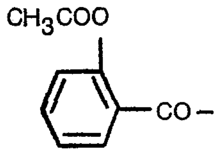 | H | 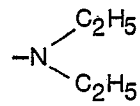 |
| 67 | 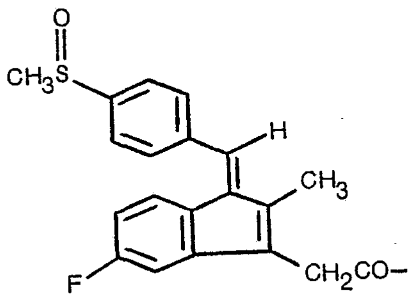 | H | 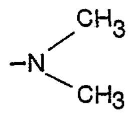 |
| 68 | 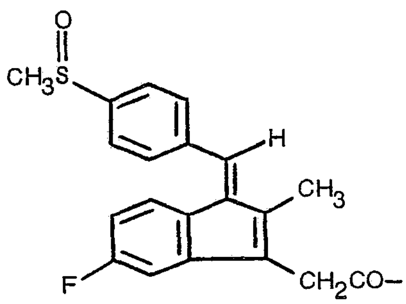 | H | 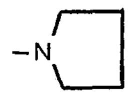 |
| 69 | 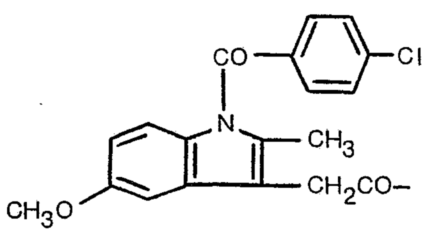 | $-CCl_3$ | 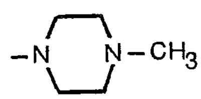 |
| 70 | 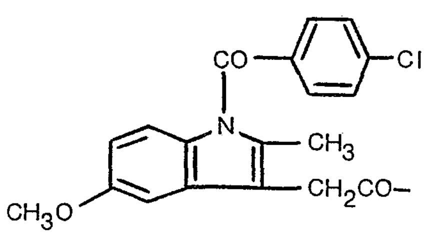 | 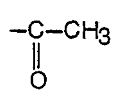 | 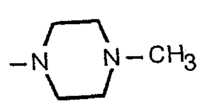 |

COMPOUNDS OF FORMULA (I) (Continued)

| Example Number | 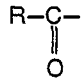 | $R_3$ | 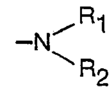 |
|---|---|---|---|
| 71 | 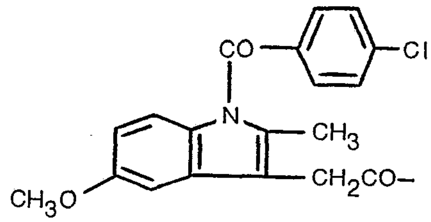 | 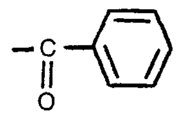 | 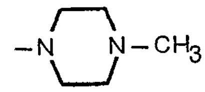 |
| 72 | 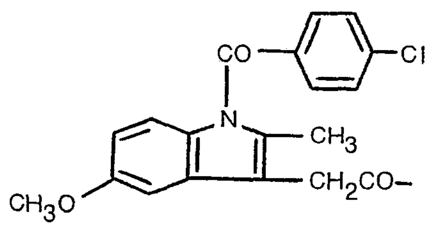 | 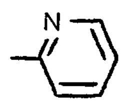 | 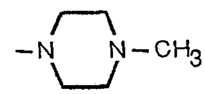 |
| 73 | 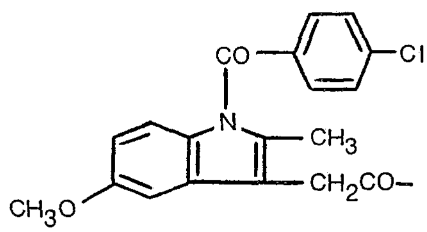 | 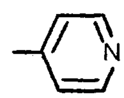 | 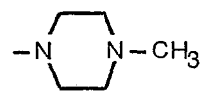 |
| 74 | 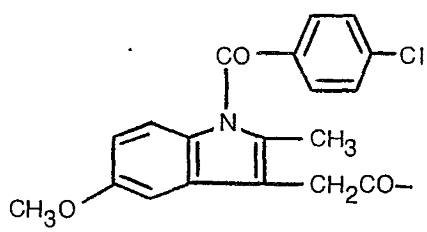 | 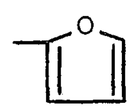 | 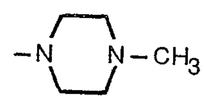 |
| 75 | 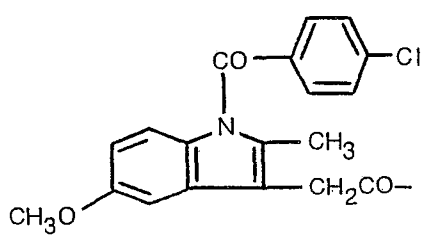 | H | 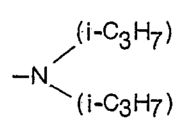 |

The anti-inflammatory activity of the prodrugs of formula (I) is evident from the results of the mouse ear assay [C. G. Van Arman, *Clin. Pharm. Ther.*, *16*, 900 (1974)] obtained when representative species of the invention were employed as the test compound. Details of the mouse ear assay are as follows:

The test animal was the male ddY strain mouse (20—25 grams each). Solutions of the test compound in acetone containing 2% croton oil (i.e., a solution containing 98 parts of acetone to 2 parts of croton oil) were prepared for varying concentrations of the test compound. Then, the test animals were anesthetized and 0.05 ml of the selected test solution was applied in 0.025 ml aliquots, one each to the anterior and posterior surface of the right ear of each mouse. Three hours after application, the mice were sacrificed via ether inhalation and both ears were removed from each mouse. Then, one circular section, 8 mm in diameter, was taken from each ear using a leather punch. The increase in weight caused by the irritant was determined by subtracting the weight of the untreated left ear section from that of the right ear section. Drug effects expressed as percent inhibition were determined at each selected concentration (molarity) of each test compound according to the following equation:

$$\%\text{inhibition} = \frac{\text{Mean Weight Increase control} - \text{Mean Weight Increase for treated}}{\text{Mean Weight Increase for control}} \times 100$$

Groups of 15 mice were treated at each dosage level of each test compound. The control group consisted of 30 mice. In the control group, the right ear of each mouse was treated with the croton oil/acetone solution described above but not containing test compound, while the left ear of each control mouse was untreated.

Indomethacin, indomethacin hydroxamic acid and the compounds of Examples 3, 4 and 5 were tested according to the mouse ear assay described above. The $ED_{50}$, that is, the dosage which was effective in reducing the weight of the ear by 50%, was obtained for each test compound from linear regression analysis of data obtained at $2 \times 10^{-2}$, $4 \times 10^{-2}$ and $8 \times 10^{-2}$ M dosage levels, i.e., the percent inhibition as calculated above was plotted against concentration and the point at which inhibition was 50% was considered the $ED_{50}$ for a given compound. The results were as follows:

| TEST COMPOUND | $ED_{50}$ (M) |
|---|---|
| Indomethacin | 0.061 |
| Indomethacin hydroxamic acid (known from US—A—3624103) | 0.096 |
| α-[1-(4′-Chlorobenzoyl)-5-methoxy-2-methylindol-3-yl]-N-morpholinomethyl)-acetohydroxamic acid | 0.054 |
| α-[1-(4′-Chlorobenzoyl)-5-methoxy-2-methylindol-3-yl]-N-[(4-methylpiperazin-1-yl)-methyl]acetohydroxamic acid | 0.027 |
| α-[1-(4′-Chlorobenzoyl)-5-methoxy-2-methylindol-3-yl]-N-(N′,N′-diethylaminomethyl)-acetohydroxamic acid | 0.041 |

The compounds of the present invention are conveniently administered to warm-blooded animals via conventional oral or topical administration, most conveniently by combining the selected compound with any suitable nontoxic pharmaceutically acceptable oral or topical inert carrier material. Such carrier materials are well known to those skilled in the art of oral and topical pharmaceutical formulations. For those not skilled in the art, reference is made to the text entitled, "REMINGTON'S PHARMACEUTICAL SCIENCES" (Fourteenth Edition), 1970. In a typical preparation for oral administration, e.g., tablet or capsule, any one of the compounds of the instant invention is combined in an anti-inflammatory effective amount with any oral nontoxic pharmaceutically acceptable inert carrier such as lactose, starch (pharmaceutical grade), dicalcium phosphate, calcium sulfate, kaolin, mannitol and powdered sugar. Additionally when required, suitable binders, lubricants, disintegrating agents and coloring agents can also be included. Typical binders include starch, gelatin, sugars such as sucrose, molasses and lactose, natural and synthetic gums such as acacia, sodium alginate, extract of Irish moss, carboxymethylcellulose, methylcellulose, polyvinylpyrrolidone, polyethylene glycol, ethylcellulose and waxes. Typical lubricants for use in these dosage forms can include, without limitation, boric acid, sodium benzoate, sodium acetate, sodium chloride, leucine and polyethylene glycol. Suitable disintegrators can include, without limitation, starch,

methylcellulose, agar, bentonite, cellulose, wood products, alginic acid, guar gum, citris pulp, carboxymethylcellulose and sodium lauryl sulfate. If desired, a conventional pharmaceutically acceptable dye can be incorporated into the dosage unit form, i.e., any of the standard FD&C dyes.

Similarly, in a typical formulation for topical application, any one of the compounds of the instant invention is combined with a topical vehicle such as triacetin, such that the active ingredient is present in an anti-inflammatory effective amount. The preparation is simply applied topically to the inflamed area, whereby the therapeutically active compound is dermally absorbed and "cleaved" to release the parent moiety at the site of inflammation.

Naturally, the therapeutic dosage range for the compounds of the instant invention will vary with the size and needs of the patient. However, generally speaking, the following dosage guidelines will suffice. On an oral basis, the therapeutic dose required for a compound of the instant invention will generally, on a molecular basis, mimic that for the parent conventional non-steroidal moiety (e.g., indomethacin, aspirin, naproxen). On a topical basis, application of a 0.01% to 2.5% concentration of a compound of the instant invention (in a suitable topical carrier material) to the site of inflammation should suffice.

**Claims for the Contracting States: BE CH DE FR GB IT LI LU NL SE**

1. A compound of the formula (I)

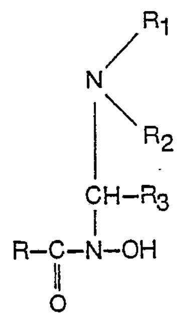

I

wherein

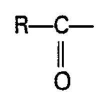

is the acyl residue of a non-steroidal anti-inflammatory agent containing a carboxylic acid function; $R_1$ and $R_2$, which can be the same or different, each represent a member selected from the group consisting of alkyl of 1 to 20 carbon atoms; aryl of 6 to 10 carbon atoms; cycloalkyl of 3 to 8 carbon atoms; aralkyl, alkaryl, alkenylaryl, and alkynylaryl, wherein the alkyl and aryl portions are defined as above and the alkenyl and alkynyl portions have 2—20 carbon atoms; and substituted derivatives of the above-defined alkyl, aryl, cycloalkyl, aralkyl, alkaryl, alkenylaryl and alkynylaryl radicals, said derivatives having one or more substituents each of which are selected from the group consisting of $C_1$—$C_8$ alkyl, $C_1$—$C_8$ alkanoyl, cyano, $C_2$—$C_9$ carbalkoxy, $C_1$—$C_8$ haloalkyl having 1 or more halo substituents, carboxyl, dialkylcarbamyl wherein the alkyl portions each contain 1 to 8 carbon atoms, and $C_1$—$C_8$ alkylsulfonyl or $R_1$ and $R_2$ are combined so that —$NR_1R_2$ represents the residue of a saturated or unsaturated heterocyclic compound; $R_3$ is selected from the group consisting of hydrogen, $R_1'$,

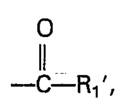

—$CH_2OCOR_1'$, —$CH_2ONO_2$, pyridyl, furyl, cyano, carbamyl, $C_2$—$C_9$ alkylcarbamyl and dialkylcarbamyl wherein the alkyl portions each contain 1 to 8 carbon atoms; $R_1'$ is any radical encompassed by the definition of $R_1$ above; or a non-toxic pharmaceutically acceptable acid addition salt or a non-toxic N-oxide thereof and/or sulfoxides in case —$NR_1R_2$ represents a sulfur containing heterocycle.

2. A compound according to Claim 1 wherein

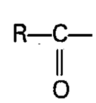

is the acyl residue of a non-steroidal anti-inflammatory agent selected from a group consisting of
(1) indomethacin;
(2) sulindac;
(3) naproxen;
(4) fenoprofan;
(5) ibuprofen;
(6) tolmetin;
(7) indoprofen;
(8) fenclozic acid;
(9) ketoprofen;
(10) alcolfenac;
(11) bucloxic acid;
(12) voltaren;
(13) cinmentacin;
(14) ibufenac;
(15) furobufen;
(16) fenclofenac;
(17) prodolic acid;
(18) pirprofen;
(19) oxoprozin;
(20) fluprofen;
(21) flurbiprofen;
(22) meclofenamic acid;
(23) flufenamic acid;
(24) cinchophen;
(25) clonixin;
(26) flutiazin;
(27) aspirin;
(28) diflunisal; and
(29) mefenamic acid.

3. A compound according to Claims 1 or 2 wherein $R_1$ and $R_2$ independently are:
$C_{1-8}$ alkyl;
phenyl;
$C_{5-6}$ cycloalkyl;
aryl $C_{1-6}$ alkyl;
$C_{1-6}$ alkyl-aryl
or a substituted derivative thereof as defined in Claim 1.

4. A compound according to Claims 1 or 2 wherein $R_1$ and $R_2$ are combined so that $—NR_1R_2$ represents the residue of a saturated or unsaturated heterocyclic compound.

5. A compound according to Claim 4 wherein the heterocyclic residue is that of a saturated monocycle containing one or more hetero atoms in the ring and optionally bearing one or more phenyl, benzyl or methyl substituents.

6. A compound according to Claim 5 wherein the heterocyclic residue is selected from the group consisting of morpholino, 1-pyrrolidinyl, 4-benzyl-1-piperazinyl, 4-methyl-1-piperazinyl, piperidino, hexamethyleneimino, 4-phenylpiperidino, 2-methyl-1-pyrazolidinyl, 3-methyl-1-imidazolidinyl, 4-benzylpiperidino and 4-phenyl-1-piperazinyl.

7. A compound according to Claim 4 wherein the heterocyclic residue is that of an unsaturated one or two ring system containing one or more double bonds and one or more ring hetero atoms, optionally substituted with one or more methyl groups.

8. A compound according to Claim 7 wherein the residue is selected from the group consisting of 1-imidazolyl, 1-pyrrolyl, 1-pyrazolyl, 1-indolinyl, 2-isoindolinyl, 1-indolyl, 2-isoindolyl, 1H-indazol-1-yl, 7-purinyl, pyrazolines, pyrrolines, and imidazolines.

9. A compound according to any one of Claims 1 to 8 wherein $R_3$ is hydrogen, alkyl, substituted alkyl having one or more halo substituents,

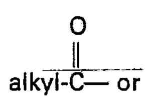 or 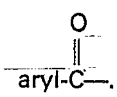

10. The compound according to Claim 1 which is selected from a group consisting of:
(1) α-[1-(4′-chlorobenzoyl)-5-methoxy-2-methylindol-3-yl]-N-(morpholinomethyl)-acetohydroxamic acid;
(2) α-[1-(4′-chlorobenzoyl)-5-methoxy-2-methylindol-3-yl]-N-(N′,N′-diethylaminomethyl)-acetohydroxamic acid;

(3) α-[1-(4'-chlorobenzoyl)-5-methoxy-2-methylindol-3-yl]-N-[(4-methylpiperazin-1-yl)methyl]-acetohydroxamic acid;

(4) α-[1-(4'-chlorobenzoyl)-5-methoxy-2-methylindol-3-yl]-N-(piperidinomethyl)acetohydroxamic acid;

(5) α-[1-(4'-chlorobenzoyl)-5-methoxy-2-methylindol-3-yl]-N-[(pyrrolidin-1-yl)methyl]-acetohydroxamic acid;

(6) α-[1-(4'-chlorobenzoyl)-5-methoxy-2-methylindol-3-yl]-N-(N',N'-dicyclohexylaminomethyl)-acetohydroxamic acid;

(7) α-[1-(4'-chlorobenzoyl)-5-methoxy-2-methylindol-3-yl]-N-[(pyrrol-1-yl)methyl]acetohydroxamic acid;

(8) α-[1-(4'-chlorobenzoyl)-5-methoxy-2-methylindol-3-yl]-N-[(indol-1-yl)methyl]acetohydroxamic acid;

(9) α-[1-(4'-chlorobenzoyl)-5-methoxy-2-methylindol-3-yl]-N-[(isoindol-2-yl)methyl]acetohydroxamic acid;

(10) α-[1-(4'-chlorobenzoyl)-5-methoxy-2-methylindol-3-yl]-N-[(purin-7-yl)methyl]acetohydroxamic acid;

(11) α-[1-(4'-chlorobenzoyl)-5-methoxy-2-methylindol-3-yl]-N-(N',N'-dimethylaminomethyl)-acetohydroxamic acid;

(12) α-[1-(4'-chlorobenzoyl)-5-methoxy-2-methylindol-3-yl]-N-(N',N'-diisopropylaminomethyl)acetohydroxamic acid;

(13) α-[1-(4'-chlorobenzoyl)-5-methoxy-2-methylindol-3-yl]-N-(N',N'-dipentylaminomethyl)-acetohydroxamic acid; and

(14) α-[1-(4'-chlorobenzoyl)-5-methoxy-2-methylindol-3-yl]-N-(N',N'-dioctylaminomethyl)-acetohydroxamic acid.

11. The compound according to Claim 1 which is selected from the group consisting of:

(1) α-{*cis*-5-fluoro-2-methyl-1-[4'-(methylsulfinyl)benzylidene]inden-3-yl}-N-(piperindinomethyl)acetohydroxamic acid;

(2) α-{*cis*-5-fluoro-2-methyl-1-[4'-(methylsulfinyl)benzylidene]inden-3-yl}-N-[(N'-benzyl-N'-phenyl)aminomethyl]acetohydroxamic acid;

(3) α-{*cis*-5-fluoro-2-methyl-1-[4'-(methylsulfinyl)benzylidene]inden-3-yl}-N-(morpholinomethyl)acetohydroxamic acid; and

(4) α-{*cis*-5-fluoro-2-methyl-1-[4'-(methylsulfinyl)benzylidene]inden-3-yl}-N-[(imidazol-1-yl)methyl]acetohydroxamic acid.

12. The compound according to Claim 1 which is α-(6-methoxynaphthalen-2-yl)-α-methyl-N-(N',N'-diethylaminomethyl)acetohydroxamic acid.

13. The compound according to Claim 1 which is selected from the group consisting of:

(1) α-methyl-α-(3-phenoxy)phenyl-N-(piperidinomethyl)acetohydroxamic acid;

(2) α-methyl-α-(3-phenoxy)phenyl-N-[(N'-benzyl-N'-phenyl)aminomethyl]acetohydroxamic acid;

(3) α-methyl-α-(3-phenoxy)phenyl-N-(morpholinomethyl)acetohydroxamic acid;

(4) α-methyl-α-(3-phenoxy)phenyl-N-[(imidazol-1-yl)methyl]acetohydroxamic acid;

(5) α-methyl-α-[4-(2'-methylpropyl)]phenyl-N-(morpholinomethyl)acetohydroxamic acid;

(6) α-methyl-α-[4-(2'-methylpropyl)]phenyl-N-(piperidinomethyl)acetohydroxamic acid;

(7) α-[1-methyl-5-(p-toluoyl)pyrrol-2-yl]-N-(N',N'-diethylaminomethyl)acetohydroxamic acid;

(8) α-methyl-α-[4-(1'-oxoisoindol-2'-yl)]phenyl-N-(piperidinomethyl)acetohydroxamic acid; and

(9) α-methyl-α-[4-(1'-oxoisoindol-2'-yl)]phenyl-N-(morpholinomethyl)acetohydroxamic acid.

14. A pharmaceutical composition comprising an anti-inflammatory effective amount of a compound of Claim 1, in combination with a nontoxic pharmaceutically acceptable inert carrier therefor.

15. A process for the preparation of a compound according to Claim 1 comprising reacting the acid chloride of a nonsteroidal anti-inflammatory agent of the formula (II)

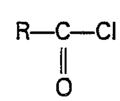

II

with hydroxylamine followed by treatment with an aldehyde of the formula

$R_3CHO$

and a secondary amine of the formula (IV)

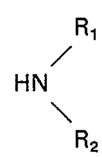

IV

wherein R, $R_1$, $R_2$ and $R_3$ are defined according to Claim 1.

**Claims for the Contracting State: AT**

1. A process for the preparation of a compound of the formula (I)

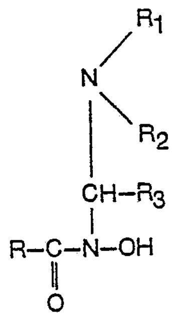

I

wherein

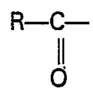

is the acyl residue of a non-steroidal anti-inflammatory agent containing a carboxylic acid function; $R_1$ and $R_2$, which can be the same or different, each represent a member selected from the group consisting of alkyl of 1 to 20 carbon atoms; aryl of 6 to 10 carbon atoms; cycloalkyl of 3 to 8 carbon atoms; aralkyl, alkaryl, alkenylaryl, and alkynylaryl, wherein the alkyl, and aryl portions are defined as above and the alkenyl and alkynyl portions have 2—20 carbon atoms; and substituted derivatives of the above-defined alkyl, aryl, cycloalkyl, aralkyl, alkaryl, alkenylaryl and alkynylaryl radicals, said derivatives having one or more substituents each of which are selected from the group consisting of $C_1$—$C_8$ alkyl, $C_1$—$C_8$ alkanoyl, cyano, $C_2$—$C_9$ carbalkoxy, $C_1$—$C_8$ haloalkyl having 1 or more halo substituents, carboxyl, dialkylcarbamyl wherein the alkyl portions each contain 1 to 8 carbon atoms, and $C_1$—$C_8$ alkylsulfonyl or $R_1$ and $R_2$ are combined so that —$NR_1R_2$ represents the residue of a saturated or unsaturated heterocyclic compound; $R_3$ is selected from the group consisting of hydrogen, $R_1'$

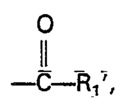

—$CH_2OCOR_1'$, —$CH_2ONO_2$, pyridyl, furyl, cyano, carbamyl, $C_2$—$C_9$ alkylcarbamyl and dialkylcarbamyl wherein the alkyl portions each contain 1 to 8 carbon atoms; $R_1'$ is any radical encompassed by the definition of $R_1'$ above; or a non-toxic pharmaceutically acceptable acid addition salt or oxide thereof, comprising reacting the acid chloride of a non-steroidal anti-inflammatory agent of the formula II

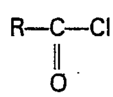

(II)

with hydroxylamine followed by treatment with an aldehyde of the formula

$R_3CHO$

and a secondary amine of the formula (IV)

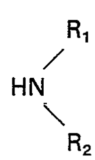

(IV)

wherein R, $R_1$, $R_2$ and $R_3$ are defined as above and, if desired, preparing a non-toxic pharmaceutically acceptable acid addition salt or a non-toxic N-oxide thereof and/or sulfoxides in case —$NR_1R_2$ represents a sulfur containing heterocycle in a manner known per se.

2. A process according to Claim 1 wherein

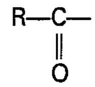

is the acyl residue of a non-steroidal anti-inflammatory agent selected from a group consisting of

(1) indomethacin;
(2) sulindac;
(3) naproxen;
(4) fenoprofan;
(5) ibuprofen;
(6) tolmetin;
(7) indoprofen;
(8) fenclozic acid;
(9) ketoprofen;
(10) alcolfenac;
(11) bucloxic acid;
(12) voltaren;
(13) cinmentacin;
(14) ibufenac;
(15) furobufen;
(16) fenclofenac;
(17) prodolic acid;
(18) pirprofen;
(19) oxoprozin;
(20) fluprofen;
(21) flurbiprofen;
(22) meclofenamic acid;
(23) flufenamic acid;
(24) cinchophen;
(25) clonixin;
(26) flutiazin;
(27) aspirin;
(28) diflunisal; and
(29) mefenamic acid.

3. A process according to Claims 1 or 2 wherein $R_1$ and $R_2$ independently are:

$C_{1-8}$ alkyl;
phenyl;
$C_{5-6}$ cycloalkyl;
aryl $C_{1-6}$ alkyl;
$C_{1-6}$ alkyl-aryl

or a substituted derivative thereof as indicated in Claim 1.

4. A process according to Claims 1 or 2 wherein $R_1$ and $R_2$ are combined so that —$NR_1R_2$ represents the residue of a saturated or unsaturated heterocyclic compound.

5. A process according to Claim 4 wherein the heterocyclic residue is that of a saturated monocycle containing one or more hetero atoms in the ring and optionally bearing one or more phenyl, benzyl or methyl substituents.

6. A process according to Claim 5 wherein the heterocyclic residue is selected from the group consisting of morpholino, 1-pyrrolidinyl, 4-benzyl-1-piperazinyl, 4-methyl-1-piperazinyl, piperidino, hexamethyleneimino, 4-phenylpiperidino, 2-methyl-1-pyrazolidinyl, 3-methyl-1-imidazolidinyl, 4-benzylpiperidino and 4-phenyl-1-piperazinyl.

7. A process according to Claim 4 wherein the heterocyclic residue is that of an unsaturated one or two ring system containing one or more double bonds and one or more ring hetero atoms, optionally substituted with one or more methyl groups.

8. A process according to Claim 7 wherein the heterocyclic residue is selected from the group consisting of 1-imidazolyl, 1-pyrrolyl, 1-pyrazolyl, 1-indolinyl, 2-isoindolinyl, 1-indolyl, 2-isoindolyl, 1H-indazol-1-yl, 7-purinyl, pyrazolines, pyrrolines, and imidazolines.

9. A process according to any one of Claims 1 to 8 wherein $R_3$ is hydrogen, alkyl, substituted alkyl having one or more halo substituents,

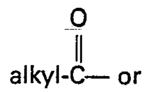 or 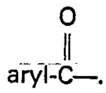.

10. The process according to Claim 1 comprising preparing a compound selected from a group consisting of:

(1) α-[1-(4′-chlorobenzoyl)-5-methoxy-2-methylindol-3-yl]-N-(morpholinomethyl)acetohydroxamic acid;

(2) α-[1-(4′-chlorobenzoyl)-5-methoxy-2-methylindol-3-yl]-N-(N′,N′-diethylaminomethyl)-acetohydroxamic acid;

(3) α-[1-(4′-chlorobenzoyl)-5-methoxy-2-methylindol-3-yl]-N-[(4-methylpiperazin-1-yl)methyl]acetohydroxamic acid;

(4) α-[1-(4′-chlorobenzoyl)-5-methoxy-2-methylindol-3-yl]-N-(piperidinomethyl)acetohydroxamic acid;

(5) α-[1-(4′-chlorobenzoyl)-5-methoxy-2-methylindol-3-yl]-N-[(pyrrolidin-1-yl)methyl]acetohydroxamic acid;

(6) α-[1-(4′-chlorobenzoyl)-5-methoxy-2-methylindol-3-yl]-N-(N′,N′-dicyclohexylaminomethyl)-acetohydroxamic acid;

(7) α-[1-(4′-chlorobenzoyl)-5-methoxy-2-methylindol-3-yl]-N-[(pyrrol-1-yl)methyl]acetohydroxamic acid;

(8) α-[1-(4′-chlorobenzoyl)-5-methoxy-2-methylindol-3-yl]-N-[(indol-1-yl)methyl]acetohydroxamic acid;

(9) α-[1-(4′-chlorobenzoyl)-5-methoxy-2-methylindol-3-yl]-N-[(isoindol-2-yl)methyl]acetohydroxamic acid;

(10) α-[1-(4′-chlorobenzoyl)-5-methoxy-2-methylindol-3-yl]-N-[(purin-7-yl)methyl]acetohydroxamic acid;

(11) α-[1-(4′-chlorobenzoyl)-5-methoxy-2-methylindol-3-yl]-N-(N′,N′-dimethylaminomethyl)-acetohydroxamic acid;

(12) α-[1-(4′-chlorobenzoyl)-5-methoxy-2-methylindol-3-yl]-N-(N′,N′-diisopropylaminomethyl)-acetohydroxamic acid;

(13) α-[1-(4′-chlorobenzoyl)-5-methoxy-2-methylindol-3-yl]-N-(N′,N′-dipentylaminomethyl)-acetohydroxamic acid; and

(14) α-[1-(4′-chlorobenzoyl)-5-methoxy-2-methylindol-3-yl]-N-(N′,N′-dioctylaminomethyl)-acetohydroxamic acid.

11. The process according to Claim 1 comprising preparing a compound selected from the group consisting of:

(1) α-{*cis*-5-fluoro-2-methyl-1-[4′-(methylsulfinyl)benzylidene]inden-3-yl}-N-(piperidinomethyl)-acetohydroxamic acid;

(2) α-{*cis*-5-fluoro-2-methyl-1-[4′-(methylsulfinyl)benzylidene]inden-3-yl}-N-[(N′-benzyl-N′-phenyl)-aminomethyl]acetohydroxamic acid;

(3) α-{*cis*-5-fluoro-2-methyl-1-[4′-(methylsulfinyl)benzylidene]inden-3-yl}-N-(morpholinomethyl)-acetohydroxamic acid; and

(4) α-{*cis*-5-fluoro-2-methyl-1-[4′-(methylsulfinyl)benzylidene]inden-3-yl}-N-[(imidazol-1-yl)methyl]acetohydroxamic acid.

12. The process according to Claim 1 comprising preparing α-(6-methoxynaphthalen-2-yl)-α-methyl-N-(N′,N′-diethylaminomethyl)acetohydroxamic acid.

13. The process according to Claim 1 comprising preparing a compound selected from the group consisting of

(1) α-methyl-α-(3-phenoxy)phenyl-N-(piperidinomethyl)acetohydroxamic acid;

(2) α-methyl-α-(3-phenoxy)phenyl-N-[(N′-benzyl-N′-phenyl)aminomethyl]acetohydroxamic acid;

(3) α-methyl-α-(3-phenoxy)phenyl-N-(morpholinomethyl)acetohydroxamic acid;

(4) α-methyl-α-(3-phenoxy)phenyl-N-[(imidazol-1-yl)methyl]acetohydroxamic acid;

(5) α-methyl-α-[4-(2′-methylpropyl)]phenyl-N-(morpholinomethyl)acetohydroxamic acid;

(6) α-methyl-α-[4-(2′-methylpropyl)]phenyl-N-(piperidinomethyl)acetohydroxamic acid;

(7) α-[1-methyl-5-(p-toluoyl)pyrrol-2-yl]-N-(N′,N′-diethylaminomethyl)acetohydroxamic acid;

(8) α-methyl-α-[4-(1′-oxoisoindol-2′-yl)]phenyl-N-(piperidinomethyl)acetohydroxamic acid; and

(9) α-methyl-α-[4-(1′-oxoisoindol-2′-yl)]phenyl-N-(morpholinomethyl)acetohydroxamic acid.

**Patentansprüche für die Vertragsstaaten: BE CH DE FR GB IT LI LU NL SE**

1. Eine Verbindung der Formel (I)

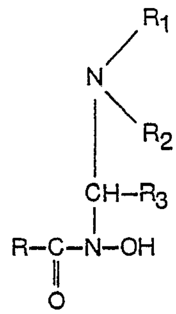

I,

worin

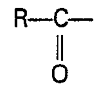

der Acylrest eines nicht-steroiden, eine Carbonsäurefunktion enthaltenden, entzündungshemmenden Mittels ist; $R_1$ und $R_2$, die gleich oder verschieden sein können, jeweils ein Glied bedeuten, das aus der Gruppe gewählt ist, welche aus Alkyl mit 1 bis 20 Kohlenstoffatomen; Aryl mit 6 bis 10 Kohlenstoffatomen; Cycloalkyl mit 3 bis 8 Kohlenstoffatomen; Aralkyl, Alkaryl, Alkenylaryl und Alkinylaryl, worin der Alkyl- und Arylabschnitt wie oben definiert sind, und die Alkenyl- und Alkinylabschnitte 2 bis 20 Kohlenstoffatome haben; und substituierten Derivaten der oben definierten Alkyl-, Aryl-, Cycloalkyl-, Aralkyl-, Alkaryl-, Alkenylaryl- und Alkinylarylreste besteht, wobei die genannten Derivate einen oder mehrere Substituenten aufweisen, von denen jeder aus der Gruppe ausgewählt ist, die aus $C_1$—$C_8$-Alkyl, $C_1$—$C_8$-Alkanoyl, Cyano, $C_2$—$C_9$-Carbalkoxy, $C_1$—$C_8$-Halogenalkyl mit einem oder mehreren Halogensubstituenten, Carboxyl, Dialkylcarbamyl, worin die Alkylabschnitte jeweils 1 bis 8 Kohlenstoffatome enthalten, und $C_1$—$C_8$-Alkylsulfonyl besteht, oder $R_1$ und $R_2$ derart kombiniert sind, daß —$NR_1R_2$ den Rest einer gesättigten oder ungesättigten, heterocyclischen Verbindung bedeutet; und $R_3$ aus der Gruppe ausgewählt ist, die aus Wasserstoff, $R_1'$,

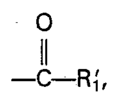

—$CH_2OCOR_1'$, —$CH_2ONO_2$, Pyridyl, Furyl, Cyano, Carbamyl, $C_2$—$C_9$-Alkylcarbamyl und Dialkylcarbamyl besteht, worin die Alkylabschnitte jeweils 1 bis 8 Kohlenstoffatome enthalten; und $R_1'$ irgendein von der Definition von $R_1$ oben umfaßter Rest ist; oder ein nicht-toxisches, pharmazeutisch annehmbares Säureadditionssalz oder ein nicht-toxisches N-Oxid davon und/oder Sulfoxide für den Fall, daß —$NR_1R_2$ einen Schwefel enthaltenden Heterocyclus bedeutet.

2. Eine Verbindung nach Anspruch 1, worin

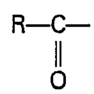

der Acylrest eines nicht-steroiden, entzündungshemmenden Mittels ist, das aus einer Gruppe ausgewählt ist, welche aus

(1) Indomethacin;
(2) Sulindac;
(3) Naproxen;
(4) Fenoprofan;
(5) Ibuprofen;
(6) Tolmetin;
(7) Indoprofen;
(8) Fenclozinsäure;
(9) Ketoprofen;
(10) Alcolfenac;
(11) Bucloxinsäure;
(12) Voltaren;
(13) Cinmentacin;
(14) Ibufenac;

(15) Furobufen;
(16) Fenclofenac;
(17) Prodolinsäure;
(18) Pirprofen;
(19) Oxoprozin;
(20) Fluprofen;
(21) Flurbiprofen;
(22) Meclofenamsäure;
(23) Flufenamsäure;
(24) Cinchophen;
(25) Clonixin;
(26) Flutiazin;
(27) Aspirin;
(28) Diflunisal; und
(29) Mefenamsäure

besteht.

3. Eine Verbindung nach Anspruch 1 oder 2, worin $R_1$ und $R_2$ unabhängig voneinander:

$C_{1-8}$-Alkyl;
Phenyl;
$C_5$—$C_6$-Cycloalkyl;
Aryl-$C_{1-6}$-alkyl; oder
$C_{1-6}$-Alkyl-aryl

sind, oder ein substituiertes Derivat davon, wie in Anspruch 1 definiert.

4. Eine Verbindung nach Anspruch 1 oder 2, worin $R_1$ und $R_2$ derart kombiniert sind, daß —$NR_1R_2$ den Rest einer gesättigten oder ungesättigten, heterocyclischen Verbindung darstellt.

5. Eine Verbindung nach Anspruch 4, worin der heterocyclische Rest ein solcher eines gesättigten Monocyclus ist, der ein oder mehrere Heteroatome im Ring enthält und gegebenenfalls einen oder mehrere Phenyl-, Benzyl- oder Methylsubstituenten trägt.

6. Eine Verbindung nach Anspruch 5, worin der heterocyclische Rest aus der Gruppe ausgewählt ist, die aus Morpholino, 1-Pyrrolidinyl, 4-Benzyl-1-piperazinyl, 4-Methyl-1-piperazinyl, Piperidino, Hexamethylenimino, 4-Phenylpiperidino, 2-Methyl-1-pyrazolidinyl, 3-Methyl-1-imidazolidinyl, 4-Benzylpiperidino und 4-Phenyl-1-piperazinyl besteht.

7. Eine Verbindung nach Anspruch 4, worin der heterocyclische Rest ein solcher eines ungesättigten, einen oder zwei Ringe aufweisenden Systems mit einer oder mehreren Doppelbindungen und einem oder mehreren Ringheteroatomen ist, das gegebenenfalls durch eine oder mehrere Methylgruppen substituiert ist.

8. Eine Verbindung nach Anspruch 7, worin der Rest aus der Gruppe ausgewählt ist, die aus 1-Imidazolyl, 1-Pyrrolyl, 1-Pyrazolyl, 1-Indolinyl, 2-Isoindolinyl, 1-Indolyl, 2-Isoindolyl, 1H-Indazol-1-yl, 7-Purinyl, Pyrazolinen, Pyrrolinen und Imidazolinen besteht.

9. Eine Verbindung nach einem der Ansprüche 1 bis 8, worin $R_3$ Wasserstoff, Alkyl, durch einen oder mehrere Halogensubstituenten substituiertes Alkyl,

$$\text{Alkyl-}\overset{\overset{\displaystyle O}{\|}}{C}\text{—} \quad \text{oder} \quad \text{Aryl-}\overset{\overset{\displaystyle O}{\|}}{C}\text{—} \quad \text{ist.}$$

10. Die Verbindung nach Anspruch 1, die aus einer Gruppe ausgewählt ist, welche aus:

(1) α-[1-(4'-Chlorbenzoyl)-5-methoxy-2-methylindol-3-yl]-N-(morpholinomethyl)-acetohydroxamsäure;

(2) α-[1-(4'-Chlorbenzoyl)-5-methoxy-2-methylindol-3-yl]-N-(N',N'-diethylaminomethyl)-acetohydroxamsäure;

(3) α-[1-(4'-Chlorbenzoyl)-5-methoxy-2-methylindol-3-yl]-N-[(4-methylpiperazin-1-yl)-methyl]acetohydroxamsäure;

(4) α-[1-(4'-Chlorbenzoyl)-5-methoxy-2-methylindol-3-yl]-N-(piperidinomethyl)acetohydroxamsäure;

(5) α-[1-(4'-Chlorbenzoyl)-5-methoxy-2-methylindol-3-yl]-N-[(pyrrolidin-1-yl)methyl]-acetohydroxamsäure;

(6) α-[1-(4'-Chlorbenzoyl)-5-methoxy-2-methylindol-3-yl]-N-(N',N'-dicyclohexylaminomethyl)acetohydroxamsäure;

(7) α-[1-(4'-Chlorbenzoyl)-5-methoxy-2-methylindol-3-yl]-N-[(pyrrol-1-yl)methyl]acetohydroxamsäure;

(8) α-[1-(4'-Chlorbenzoyl)-5-methoxy-2-methylindol-3-yl]-N-[(indol-1-yl)methyl]acetohydroxamsäure;

(9) α-[1-(4'-Chlorbenzoyl)-5-methoxy-2-methylindol-3-yl]-N-[(isoindol-2-yl)methyl]acetohydroxamsäure;

(10) α-[1-(4'-Chlorbenzoyl)-5-methoxy-2-methylindol-3-yl]-N-[(purin-7-yl)methyl]acetohydroxamsäure;

(11) α-[1-(4′-Chlorbenzoyl)-5-methoxy-2-methylindol-3-yl]-N-(N′,N′-dimethylaminomethyl)acetohydroxamsäure;

(12) α-[1-(4′-Chlorbenzoyl)-5-methoxy-2-methylindol-3-yl]-N-(N′,N′-diisopropylaminomethyl)acetohydroxamsäure;

(13) α-[1-(4′-Chlorbenzoyl)-5-methoxy-2-methylindol-3-yl]-N-(N′,N′-dipentylaminomethyl)acetohydroxamsäure; und

(14) α-[1-(4′-Chlorbenzoyl)-5-methoxy-2-methylindol-3-yl]-N-(N′,N′-dioctylaminomethyl)acetohydroxamsäure

besteht.

11. Die Verbindung nach Anspruch 1, welche aus der Gruppe ausgewählt ist, die aus:

(1) α-{cis-5-Fluor-2-methyl-1-[4′-(methylsulfinyl)benzyliden]inden-3-yl}-N-(piperidinomethyl)acetohydroxamsäure;

(2) α-{cis-5-Fluor-2-methyl-1-[4′-(methylsulfinyl)benzyliden]inden-3-yl}-N-[(N′-benzyl-N′-phenyl)-aminomethyl]acetohydroxamsäure;

(3) α-{cis-5-Fluor-2-methyl-1-[4′-(methylsulfinyl)benzyliden]inden-3-yl}-N-(morpholinomethyl)acetohydroxamsäure; und

(4) α-{cis-5-Fluor-2-methyl-1-[4′-(methylsulfinyl)benzyliden]inden-3-yl}-N-[(imidazol-1-yl)methyl]-acetohydroxamsäure

besteht.

12. Die Verbindung nach Anspruch 1, welche α-(6-Methoxynaphthalin-2-yl)-α-methyl-N-(N′,N′-diethylaminomethyl)acetohydroxamsäure ist.

13. Die Verbindung nach Anspruch 1, welche aus der Gruppe ausgewählt ist, die aus

(1) α-Methyl-α-(3-phenoxy)phenyl-N-(piperidinomethyl)acetohydroxamsäure;

(2) α-Methyl-α-(3-phenoxy)phenyl-N-[(N′-benzyl-N′-phenyl)aminomethyl]acetohydroxamsäure;

(3) α-Methyl-α-(3-phenoxy)phenyl-N-(morpholinomethyl)acetohydroxamsäure;

(4) α-Methyl-α-(3-phenoxy)phenyl-N-[(imidazol-1-yl)methyl]acetohydroxamsäure;

(5) α-Methyl-α-[4-(2′-methylpropyl)]phenyl-N-(morpholinomethyl)acetohydroxamsäure;

(6) α-Methyl-α-[4-(2′-methylpropyl)]phenyl-N-(piperidinomethyl)acetohydroxamsäure;

(7) α-[1-Methyl-5-(p-toluoyl)pyrrol-2-yl]-N-(N′,N′-diethylaminomethyl)acetohydroxamsäure;

(8) α-Methyl-α-[4-(1′-oxoisoindol-2′-yl)]phenyl-N-(piperidinomethyl)acetohydroxamsäure; und

(9) α-Methyl-α-[4-(1′-oxoisoindol-2′-yl)]phenyl-N-(morpholinomethyl)acetohydroxamsäure

besteht.

14. Eine pharmazeutische Zusammensetzung, enthaltend eine entzündungshemmend wirkende Menge einer Verbindung des Anspruchs 1, in Kombination mit einem nicht-toxischen, pharmazeutisch annehmbaren, inerten Träger dafür.

15. Ein Verfahren für die Herstellung einer Verbindung nach Anspruch 1, umfassend die Umsetzung des Säurechlorids eines nicht-steroiden, entzündungshemmenden Mittels der Formel (II)

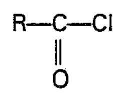

II

mit Hydroxylamin und anschließende Behandlung mit einem Aldehyd der Formel

$$R_3CHO$$

und einem sekundären Amin der Formel (IV)

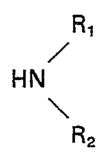

(IV),

worin R, $R_1$, $R_2$ und $R_3$ gemäß Anspruch 1 definiert sind.

**Patentansprüche für den Vertragsstaat: AT**

1. Ein Verfahren zur Herstellung einer Verbindung der Formel (I)

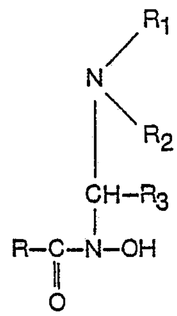

I,

worin

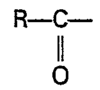

der Acylrest eines nicht-steroiden, eine Carbonsäurefunktion enthaltenden, entzündungshemmenden Mittels ist; $R_1$ und $R_2$, die gleich oder verschieden sein können, jeweils ein Glied bedeuten, das aus der Gruppe gewählt ist, welche aus Alkyl mit 1 bis 20 Kohlenstoffatomen; Aryl mit 6 bis 10 Kohlenstoffatomen; Cycloalkyl mit 3 bis 8 Kohlenstoffatomen; Aralkyl, Alkaryl, Alkenylaryl und Alkinylaryl, worin der Alkyl- und Arylabschnitt wie oben definiert sind, und die Alkenyl- und Alkinylabschnitte 2 bis 20 Kohlenstoffatome haben; und substituierten Derivaten der oben definierten Alkyl-, Aryl-, Cycloalkyl-, Aralkyl-, Alkaryl-, Alkenylaryl- und Alkinylarylreste besteht, wobei die genannten Derivate einen oder mehrere Substituenten aufweisen, von denen jeder aus der Gruppe ausgewählt ist, die aus $C_1$—$C_8$-Alkyl, $C_1$—$C_8$-Alkanoyl, Cyano, $C_2$—$C_9$-Carbalkoxy, $C_1$—$C_8$-Halogenalkyl mit einem oder mehreren Halogensubstituenten, Carboxyl, Dialkylcarbamyl, worin die Alkylabschnitte jeweils 1 bis 8 Kohlenstoffatome enthalten, und $C_1$—$C_8$-Alkylsulfonyl besteht, oder $R_1$ und $R_2$ derart kombiniert sind, daß —$NR_1R_2$ den Rest einer gesättigten oder ungesättigten, heterocyclischen Verbindung bedeutet; und $R_3$ aus der Gruppe ausgewählt ist, die aus Wasserstoff, $R_1'$,

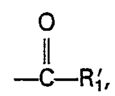

—$CH_2OCOR_1'$, —$CH_2ONO_2$, Pyridyl, Furyl, Cyano, Carbamyl, $C_2$—$C_9$-Alkylcarbamyl und Dialkylcarbamyl besteht, worin die Alkylabschnitte jeweils 1 bis 8 Kohlenstoffatome enthalten; und $R_1'$ irgendein von der Definition von $R_1$ oben umfaßter Rest ist; oder eines nicht-toxische, pharmazeutisch annehmbaren Säureadditionssalzes oder N-Oxides davon, umfassend die Umsetzung des Säurechlorids eines nicht-steroiden, entzündungshemmenden Mittels der Formel (II)

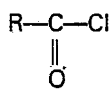

II

mit Hydroxylamin und anschließende Behandlung mit einem Aldehyd der Formel

$R_3CHO$

und einem sekundären Amin der Formel (IV)

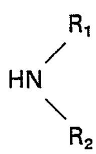

(IV),

worin R, $R_1$, $R_2$ und $R_3$ wie oben definiert sind, und gewünschtenfalls das Herstellen in an sich bekannter Weise eines nicht-toxischen, pharmazeutisch annehmbaren Säureadditionssalzes oder eines nicht-toxischen N-Oxides davon, und/oder von Sulfoxiden für den Fall, daß —$NR_1R_2$ einen Schwefel enthaltenden Heterocyclus bedeutet.

2. Ein Verfahren nach Anspruch 1, worin

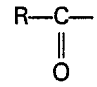

der Acylrest eines nicht-steroiden, entzündungshemmenden Mittels ist, das aus einer Gruppe ausgewählt ist, welche aus

(1) Indomethacin;
(2) Sulindac;
(3) Naproxen;
(4) Fenoprofan;
(5) Ibuprofen;
(6) Tolmetin;
(7) Indoprofen;
(8) Fenclozinsäure;
(9) Ketoprofen;
(10) Alcolfenac;
(11) Bucloxinsäure;
(12) Voltaren;
(13) Cinmentacin;
(14) Ibufenac;
(15) Furobufen;
(16) Fenclofenac;
(17) Prodolinsäure;
(18) Pirprofen;
(19) Oxoprozin;
(20) Fluprofen;
(21) Flurbiprofen;
(22) Meclofenamsäure;
(23) Flufenamsäure;
(24) Cinchophen;
(25) Clonixin;
(26) Flutiazin;
(27) Aspirin;
(28) Diflunisal; und
(29) Mefenamsäure

besteht.

3. Ein Verfahren nach Anspruch 1 oder 2, worin $R_1$ und $R_2$ unabhängig voneinander:

$C_{1-8}$-Alkyl;
Phenyl;
$C_5$—$C_6$-Cycloalkyl;
Aryl-$C_{1-6}$-alkyl; oder
$C_{1-6}$-Alkyl-aryl

sind, oder ein substituiertes Derivat davon, wie in Anspruch 1 angegeben.

4. Ein Verfahren nach Anspruch 1 oder 2, worin $R_1$ und $R_2$ derart kombiniert sind, daß —$NR_1R_2$ den Rest einer gesättigten oder ungesättigten, heterocyclischen Verbindung darstellt.

5. Ein Verfahren nach Anspruch 4, worin der heterocyclische Rest ein solcher eines gesättigten Monocyclus ist, der ein oder mehrere Heteroatome im Ring enthält und gegebenenfalls einen oder mehrere Phenyl-, Benzyl- oder Methylsubstituenten trägt.

6. Ein Verfahren nach Anspruch 5, worin der heterocyclische Rest aus der Gruppe ausgewählt ist, die aus Morpholino, 1-Pyrrolidinyl, 4-Benzyl-1-piperazinyl, 4-Methyl-1-piperazinyl, Piperidino, Hexamethylenimino, 4-Phenylpiperidino, 2-Methyl-1-pyrazolidinyl, 3-Methyl-1-imidazolidinyl, 4-Benzylpiperidino und 4-Phenyl-1-piperazinyl besteht.

7. Ein Verfahren nach Anspruch 4, worin der heterocyclische Rest ein solcher eines ungesättigten, einen oder zwei Ringe aufweisenden Systems mit einer oder mehreren Doppelbindungen und einem oder mehreren Ringheteroatomen ist, das gegebenenfalls durch eine oder mehrere Methylgruppen substituiert ist.

8. Ein Verfahren nach Anspruch 7, worin der heterocyclische Rest aus der Gruppe ausgewählt ist, die aus 1-Imidazolyl, 1-Pyrrolyl, 1-Pyrazolyl, 1-Indolinyl, 2-Isoindolinyl, 1-Indolyl, 2-Isoindolyl, 1H-Indazol-1-yl, 7-Purinyl, Pyrazolinen, Pyrrolinen und Imidazolinen besteht.

9. Ein Verfahren nach einem der Ansprüche 1 bis 8, worin $R_3$ Wasserstoff, Alkyl, durch einen oder mehrere Halogensubstituenten substituiertes Alkyl,

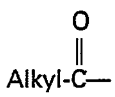 oder 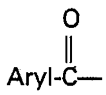 ist.

10. Das Verfahren nach Anspruch 1, umfassend die Herstellung einer Verbindung, die aus einer Gruppe ausgewählt ist, welche aus:

(1) α-[1-(4'-Chlorbenzoyl)-5-methoxy-2-methylindol-3-yl]-N-(morpholinomethyl)-acetohydroxamsäure;

(2) α-[1-(4'-Chlorbenzoyl)-5-methoxy-2-methylindol-3-yl]-N-(N',N'-diethylaminomethyl)-acetohydroxamsäure;

(3) α-[1-(4'-Chlorbenzoyl)-5-methoxy-2-methylindol-3-yl]-N-[(4-methylpiperazin-1-yl)-methyl]acetohydroxamsäure;

(4) α-[1-(4'-Chlorbenzoyl)-5-methoxy-2-methylindol-3-yl]-N-(piperidinomethyl)acetohydroxamsäure;

(5) α-[1-(4'-Chlorbenzoyl)-5-methoxy-2-methylindol-3-yl]-N-[(pyrrolidin-1-yl)methyl]-acetohydroxamsäure;

(6) α-[1-(4'-Chlorbenzoyl)-5-methoxy-2-methylindol-3-yl]-N-(N',N'-dicyclohexylaminomethyl)acetohydroxamsäure;

(7) α-[1-(4'-Chlorbenzoyl)-5-methoxy-2-methylindol-3-yl]-N-[(pyrrol-1-yl)methyl]acetohydroxamsäure;

(8) α-[1-(4'-Chlorbenzoyl)-5-methoxy-2-methylindol-3-yl]-N-[(indol-1-yl)methyl]acetohydroxamsäure;

(9) α-[1-(4'-Chlorbenzoyl)-5-methoxy-2-methylindol-3-yl]-N-[(isoindol-2-yl)methyl]acetohydroxamsäure;

(10) α-[1-(4'-Chlorbenzoyl)-5-methoxy-2-methylindol-3-yl]-N-[(purin-7-yl)methyl]acetohydroxamsäure;

(11) α-[1-(4'-Chlorbenzoyl)-5-methoxy-2-methylindol-3-yl]-N-(N',N'-dimethylaminomethyl)acetohydroxamsäure;

(12) α-[1-(4'-Chlorbenzoyl)-5-methoxy-2-methylindol-3-yl]-N-(N',N'-diisopropylaminomethyl)acetohydroxamsäure;

(13) α-[1-(4'-Chlorbenzoyl)-5-methoxy-2-methylindol-3-yl]-N-(N',N'-dipentylaminomethyl)acetohydroxamsäure; und

(14) α-[1-(4'-Chlorbenzoyl)-5-methoxy-2-methylindol-3-yl]-N-(N',N'-dioctylaminomethyl)acetohydroxamsäure

besteht.

11. Das Verfahren nach Anspruch 1, umfassend die Herstellung einer Verbindung, die aus der Gruppe ausgewählt ist, die aus:

(1) α-{cis-5-Fluor-2-methyl-1-[4'-(methylsulfinyl)benzyliden]inden-3-yl}-N-(piperidinomethyl)acetohydroxamsäure;

(2) α-{cis-5-Fluor-2-methyl-1-[4'-(methylsulfinyl)benzyliden]inden-3-yl}-N-[(N'-benzyl-N'-phenyl)-aminomethyl]acetohydroxamsäure;

(3) α-{cis-5-Fluor-2-methyl-1-[4'-(methylsulfinyl)benzyliden]inden-3-yl}-N-(morpholinomethyl)acetohydroxamsäure; und

(4) α-{cis-5-Fluor-2-methyl-1-[4'-(methylsulfinyl)benzyliden]inden-3-yl}-N-[(imidazol-1-yl)methyl]-acetohydroxamsäure

besteht.

12. Das Verfahren nach Anspruch 1, umfassend die Herstellung von α-(6-Methoxynaphthalin-2-yl)-α-methyl-N-(N',N'-diethylaminomethyl)acetohydroxamsäure.

13. Das Verfahren nach Anspruch 1, umfassend die Herstellung einer Verbindung, die aus der Gruppe ausgewählt ist, die aus:

(1) α-Methyl-α-(3-phenoxy)phenyl-N-(piperidinomethyl)acetohydroxamsäure;

(2) α-Methyl-α-(3-phenoxy)phenyl-N-[(N'-benzyl-N'-phenyl)aminomethyl]acetohydroxamsäure;

(3) α-Methyl-α-(3-phenoxy)phenyl-N-(morpholinomethyl)acetohydroxamsäure;

(4) α-Methyl-α-(3-phenoxy)phenyl-N-[(imidazol-1-yl)methyl]acetohydroxamsäure;

(5) α-Methyl-α-[4-(2'-methylpropyl)]phenyl-N-(morpholinomethyl)acetohydroxamsäure;

(6) α-Methyl-α-[4-(2'-methylpropyl)]phenyl-N-(piperidinomethyl)acetohydroxamsäure;

(7) α-[1-Methyl-5-(p-toluoyl)pyrrol-2-yl]-N-(N',N'-diethylaminomethyl)acetohydroxamsäure;

(8) α-Methyl-α-[4-(1'-oxoisoindol-2'-yl)]phenyl-N-(piperidinomethyl)acetohydroxamsäure; und

(9) α-Methyl-α-[4-(1'-oxoisoindol-2'-yl)]phenyl-N-(morpholinomethyl)acetohydroxamsäure

besteht.

**Revendications pour les Etats contractants: BE CH DE FR GB IT LI LU NL SE**

1. Composé de formule (I)

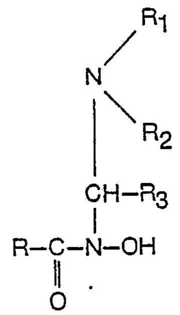

I

où

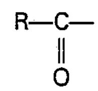

est le résidu acyle de n'importe quel agent anti-inflammatoire non stéroïdique contenant une fonction acide carboxylique; R1 et R2, qui peuvent être semblables ou différents, représentent chacun un membre choisi dans le groupe constitué par alcoyle en C1 à C20; aryle en C6 à C10; cycloalcoyle en C3 à C8; aralcoyle, alcaryle, alcénylaryle, et alcynylaryle, où les fractions alcoyle et aryle sont définies comme ci-dessus et les fractions alcényle et alcynyle sont en C2 à C20; et les dérivés substitués des radicaux alcoyle, aryle, cycloalcoyle, aralcoyle, alcaryle, alcénylaryle et alcynylaryle définis ci-dessus, lesdits dérivés ayant un ou plusieurs substituants qui sont chacun choisi dans le groupe constitué par alcoyle en C1 à C8, alcanoyle en C1 à C8, cyano, carbalcoxy en C2 à C9, haloalcoyle en C1 à C8 ayant un ou plusieurs substituants halo, carboxyle, dialcoylcarbamyle où les fractions alcoyle sont chacune en C1 à C8, et alcoylsulfonyle en C1 à C8; ou R1 et R2 sont combinés de manière que —NR1R2 représente le résidu d'un composé hétérocyclique saturé ou non saturé, R3 est choisi dans le groupe constitué par hydrogène,

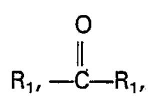 $—CH_2OCOR_1$, $—CH_2ONO_2$,

pyridyle, furyle, cyano, carbamyle, alcoyle en C2 à C9-carbamyle et dialcoyl-carbamyle où les fractions alcoyle contiennent chacune de 1 à 8 atomes de carbone; R1 est tout radical compris dans la définition de R1 ci-dessus; ou d'un de ses sels d'addition d'acides ou N-oxyde non toxique pharmaceutiquement acceptable, et/ou des sulfoxydes au cas où —NR1R2 représente un hétérocycle contenant du soufre.

2. Composé selon la revendication 1 où

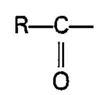

est le résidu acyle d'un agent anti-inflammatoire non stéroïdique choisi dans un groupe constitué par

(1) indométhacine;
(2) sulindac;
(3) naproxène;
(4) fenoprofan;
(5) ibuprofène;
(6) tolmétine;
(7) indoprofène;
(8) Acide fenclozique
(9) cétoprofène;
(10) alcolfénac;
(11) Acide bucloxique
(12) voltarène;
(13) cinmentacine;
(14) ibufénac;
(15) furobufène;
(16) fenclofénac;
(17) Acide prodolique

(18) pirprofène;
(19) oxoprozine;
(20) fluprofène;
(21) flurbiprofène;
(22) Acide méclofénamique
(23) Acide flufénamique
(24) cinchophène;
(25) clonixine;
(26) flutiazine;
(27) aspirine;
(28) diflunisal; et
(29) Acide méfénamique.

3. Composé selon les revendications 1 et 2 où R1 et R2 représentent indépendamment:
un alcoyle en C1 à C8;
un phényle;
un cycloalcoyle en C5 à C6;
un aryl-alcoyle en C1 à C6;
un alcoyle en C1 à C6-aryle
ou un de leurs dérivés substitués tels que définis dans la revendication 1.

4. Composé selon les revendications 1 et 2 où R1 et R2 sont combinés de manière telle que —NR1R2 représente le résidu d'un composé hétérocyclique saturé ou non saturé.

5. Composé selon la revendication 4 où le résidu hétérocyclique est celui d'un monocycle saturé contenant un ou plusieurs hétéroatomes dans le noyau et portant éventuellement un ou plusieurs substituants phényle, benzyle ou méthyle.

6. Composé selon la revendication 5 où le résidu hétérocyclique est choisi dans le groupe constitué par morpholino, 1-pyrrolidinyle, 4-benzyl-1-pipérazinyle, 4-méthyl-1-pipérazinyle, pipéridino, hexaméthylèneimino, 4-phénylpipéridino, 2-méthyl-1-pyrazolidinyle, 3-méthyl-1-imidazolidinyle, 4-benzylpipéridino et 4-phényl-1-pipérazinyle.

7. Composé selon la revendication 4 où le résidu hétérocyclique est celui d'un système mono- ou bicyclique non saturé contenant une ou plusieurs doubles liaisons et un ou plusieurs hétéroatomes cycliques, éventuellement substitué par un ou plusieurs groupes méthyle.

8. Composé selon la revendication 7 où le résidu est choisi dans le groupe constitué par 1-imidazolyle, 1-pyrrolyle, 1-pyrazolyle, 1-indolinyle, 2-isoindolinyle, 1-indolyle, 2-isoindolyle, 1H-indazol-1-yle, 7-purinyle, les pyrazolines, pyrrolines et imidazolines.

9. Composé selon l'une quelconque des revendications 1 à 8 où R3 est un hydrogène, un alcoyle, un alcoyle substitué ayant ou un plusieurs substituants halo, un

$$\text{alcoyl-}\overset{\overset{\displaystyle O}{\|}}{C}\text{—} \quad \text{ou} \quad \text{aryl-}\overset{\overset{\displaystyle O}{\|}}{C}\text{—}.$$

10. Composé selon la revendication 1 qui est choisi dans un groupe constitué par:
(1) Acide α-[1-(4'-chlorobenzoyl)-5-méthoxy-2-méthylindol-3-yl]-N-(morpholinométhyl)-acétohydroxamique
(2) Acide α-[1-(4'-chlorobenzoyl)-5-méthoxy-2-méthylindol-3-yl]-N-(N',N'-diéthylaminométhyl)acéto-hydroxamique
(3) Acide α-[1-(4'-chlorobenzoyl)-5-méthoxy-2-méthylindol-3-yl]-N-[(4-méthylpipérazin-1-yl)méthyl]-acétohydroxamique
(4) Acide α-[1-(4'-chlorobenzoyl)-5-méthoxy-2-méthylindol-3-yl]-N-(pipéridinométhyl)acéto-hydroxamique hydroxamique
(5) Acide α-[1-(4'-chlorobenzoyl)-5-méthoxy-2-méthylindol-3-yl]-N-[(pyrrolidin-1-yl)méthyl]acéto-hydroxamique
(6) Acide α-[1-(4'-chlorobenzoyl)-5-méthoxy-2-méthylindol-3-yl]-N-(N',N'-dicyclohexylaminométhyl)-acétohydroxamique
(7) Acide α-[1-(4'-chlorobenzoyl)-5-méthoxy-2-méthylindol-3-yl]-N-[(pyrrol-1-yl)méthyl]acéto-hydroxamique
(8) Acide α-[1-(4'-chlorobenzoyl)-5-méthoxy-2-méthylindol-3-yl]-N-[(indol-1-yl)méthyl]acéto-hydroxamique
(9) Acide α-[1-(4'-chlorobenzoyl)-5-méthoxy-2-méthylindol-3-yl]-N-[(isoindol-2-yl)méthyl]acéto-hydroxamique
(10) Acide α-[1-(4'-chlorobenzoyl)-5-méthoxy-2-méthylindol-3-yl]-N-[(purin-7-yl)méthyl]acéto-hydroxamique
(11) Acide α-[1-(4'-chlorobenzoyl)-5-méthoxy-2-méthylindol-3-yl]-N-(N',N'-diméthylaminométhyl)-acétohydroxamique

(12) Acide α-[1-(4'-chlorobenzoyl)-5-méthoxy-2-méthylindol-3-yl]-N-(N',N'-diisopropylaminométhyl)-acétohydroxamique

(13) Acide α-[1-(4'-chlorobenzoyl)-5-méthoxy-2-méthylindol-3-yl]-N-(N',N'-dipentylaminométhyl)-acétohydroxamique

(14) Acide α-[1-(4'-chlorobenzoyl)-5-méthoxy-2-méthylindol-3-yl]-N-(N',N'-dioctylaminométhyl)acétohydroxamique.

11. Composé selon la revendication 1 qui est choisi dans le groupe constitué par:

(1) Acide α-{*cis*-5-fluoro-2-méthyl-1-[4'-(méthylsulfinyl)benzylidène]indén-3-yl}-N-(pipéridinométhyl)-acétohydroxamique

(2) Acide α-{*cis*-5-fluoro-2-méthyl-1-[4'-(méthylsulfinyl)benzylidène]indén-3-yl}-N-[(N'-benzyl-N'-phényl)aminométhyl]acétohydroxamique

(3) Acide α-{*cis*-5-fluoro-2-méthyl-1-[4'-(méthylsulfinyl)benzylidène]indén-3-yl}-N-(morpholinométhyl)acétohydroxamique

(4) Acide α-{*cis*-5-fluoro-2-méthyl-1-[4'-(méthylsulfinyl)benzylidène]indén-3-yl}-N-[(imidazol-1-yl)-méthyl]acétohydroxamique.

12. Composé selon la revendication 1 qui est Acide α-(6-méthoxynaphtalén-2-yl)-α-méthyl-N-(N',N'-diéthylaminométhyl)acétohydroxamique.

13. Composé selon la revendication 1 qui est choisi dans le groupe constitué par:

(1) Acide α-méthyl-α-(3-phénoxy)phényl-N-(pipéridinométhyl)acétohydroxamique

(2) Acide α-méthyl-α-(3-phénoxy)phényl-N-[(N'-benzyl-N'-phényl)aminométhyl]acétohydroxamique

(3) Acide α-méthyl-α-(3-phénoxy)phényl-N-(morpholinométhyl)acétohydroxamique

(4) Acide α-méthyl-α-(3-phénoxy)phényl-N-[(imidazol-1-yl)méthyl]acétohydroxamique

(5) Acide α-méthyl-α-[4-(2'-méthylpropyl)]phényl-N-(morpholinométhyl)acétohydroxamique

(6) Acide α-méthyl-α-[4-(2'-méthylpropyl)]phényl-N-(pipéridinométhyl)acétohydroxamique

(7) Acide α-[1-méthyl-5-(p-toluoyl)pyrrol-2-yl]-N-(N',N'-diéthylaminométhyl)acétohydroxamique

(8) Acide α-méthyl-α-[4-(1'oxoisoindol-2'-yl)]phényl-N-(pipéridinométhyl)acétohydroxamique

(9) Acide α-méthyl-α-[4-(1'-oxoisoindol-2'-yl)]phényl-N-(morpholinométhyl)acétohydroxamique.

14. Composition pharmaceutique comprenant une quantité efficace comme anti-inflammatoire d'un composé de la revendication 1, en combinaison avec un support inerte non toxique pharmaceutiquement acceptable à cet effet.

15. Procédé de préparation d'un composé selon la revendication 1 dans lequel on fait réagir le chlorure d'acide d'un agent anti-inflammatoire non stéroïdique de formule (II)

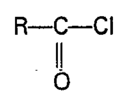

II

avec de l'hydroxylamine puis on traite avec un aldéhyde de formule

$$R_3CHO$$

et une amine secondaire de formule (IV)

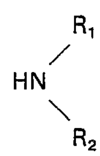

IV

où R, R1, R2 et R3 sont définis selon la revendication 1.

**Revendications pour l'Etat contractant: AT**

1. Procédé de préparation d'un composé de formule (I)

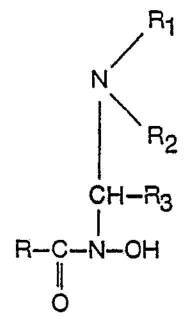

I

où

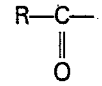

est le résidu acyle de n'importe quel agent anti-inflammatoire non stéroïdique contenant une fonction acide carboxylique; R1 et R2, qui peuvent être semblables ou différents, représentent chacun un membre choisi dans le groupe constitué par alcoyle en C1 à C20; aryle en C6 à C10; cycloalcoyle en C3 à C8; aralcoyle, alcaryle, alcénylaryle, et alcynylaryle, où les fractions alcoyle et aryle sont définies comme ci-dessus et les fractions alcényle et alcynyle sont en C2 à C20; et les dérivés substitués des radicaux alcoyle, aryle, cycloalcoyle, aralcoyle, alcaryle, alcénylaryle et alcynylaryle définis ci-dessus, lesdits dérivés ayant un ou plusieurs substituants qui sont chacun choisi dans le groupe constitué par alcoyle en C1 à C8, alcanoyle en C1 à C8, cyano, carbalcoxy en C2 à C9, haloalcoyle en C1 à C8 ayant un ou plusieurs substituants halo, carboxyle, dialcoylcarbamyle où les fractions alcoyle sont chacune en C1 à C8, et alcoylsulfonyle en C1 à C8; ou R1 et R2 sont combinés de manière que —NR1R2 représente le résidu d'un composé hétérocyclique saturé ou non saturé, R3 est choisi dans le groupe constitué par hydrogène,

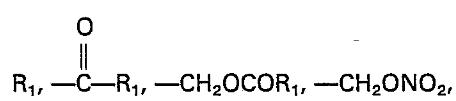

pyridyle, furyle, cyano, carbamyle, alcoyle en C2 à C9-carbamyle et dialcoyl-carbamyle où les fractions alcoyle contiennent chacune de 1 à 8 atomes de carbone; R1 est tout radical compris dans la définition de R1 ci-dessus; ou d'un de ses sels d'addition d'acides ou N-oxyde non toxique pharmaceutiquement acceptable, dans lequel on fait réagir le chlorure d'acide d'un agent anti-inflammatoire non stéroïdique de formule (II)

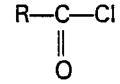

II

avec de l'hydroxylamine puis on traite avec un aldéhyde de formule

$R_3CHO$

et une amine secondaire de formule (IV)

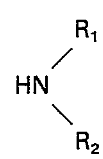

IV

où R, R1, R2 et R3 sont définis comme ci-dessus et, si on le désire, on prépare un de ses sels d'addition d'acides non toxiques pharmaceutiquement acceptables ou N-oxydes non toxiques et/ou sulfoxydes lorsque —NR1R2 représente un hétérocycle contenant du soufre de façon connue.

2. Procédé selon la revendication 1 où

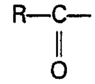

est le résidu acyle d'un agent anti-inflammatoire non stéroïdique choisi dans un groupe constitué par
(1) indométhacine;
(2) sulindac;
(3) naproxène;
(4) fenoprofan;
(5) ibuprofène;
(6) tolmétine;
(7) indoprofène;
(8) Acide fenclozique
(9) cétoprofène;
(10) alcolfénac;
(11) Acide bucloxique
(12) voltarène;
(13) cinmentacine;
(14) ibufénac;
(15) furobufène;
(16) fenclofénac;
(17) Acide prodolique
(18) pirprofène;
(19) oxoprozine;
(20) fluprofène;
(21) flurbiprofène;
(22) Acide méclofénamique
(23) Acide flufénamique
(24) cinchophène;
(25) clonixine;
(26) flutiazine;
(27) aspirine;
(28) diflunisal; et
(29) Acide méfénamique.

3. Procédé selon les revendications 1 et 2 où R1 et R2 représentent indépendamment:
un alcoyle en C1 à C8;
un phényle;
un cycloalcoyle en C5 à C6;
un aryl-alcoyle en C1 à C6;
un alcoyle en C1 à C6-aryle
ou un de leurs dérivés substitués tels que définis dans la revendication 1.

4. Procédé selon les revendications 1 et 2 où R1 et R2 sont combinés de manière telle que —NR1R2 représente le résidu d'un composé hétérocyclique saturé ou non saturé.

5. Procédé selon la revendication 4 où le résidu hétérocyclique est celui d'un monocycle saturé contenant un ou plusieurs hétéroatomes dans le noyau et portant éventuellement un ou plusieurs substituants phényle, benzyle ou méthyle.

6. Procédé selon la revendication 5 où le résidu hétérocyclique est choisi dans le groupe constitué par morpholino, 1-pyrrolidinyle, 4-benzyl-1-pipérazinyle, 4-méthyl-1-pipérazinyle, pipéridino, hexaméthylèneimino, 4-phénylpipéridino, 2-méthyl-1-pyrazolidinyle, 3-méthyl-1-imidazolidinyle, 4-benzylpipéridino et 4-phényl-1-pipérazinyle.

7. Procédé selon la revendication 4 où le résidu hétérocyclique est celui d'un système mono- ou bicyclique non saturé contenant une ou plusieurs doubles liaisons et un ou plusieurs hétéroatomes cycliques, éventuellement substitué par un ou plusieurs groupes méthyle.

8. Procédé selon la revendication 7 où le résidu est choisi dans le groupe constitué par 1-imidazolyle, 1-pyrrolyle, 1-pyrazolyle, 1-indolinyle, 2-isoindolinyle, 1-indolyle, 2-isoindolyle, 1H-indazol-1-yle, 7-purinyle, les pyrazolines, pyrrolines et imidazolines.

9. Procédé selon l'une quelconque des revendications 1 à 8 où R3 est un hydrogène, un alcoyle, un alcoyle substitué ayant un ou plusieurs substituants halo, un

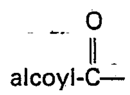 ou 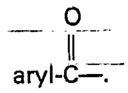

10. Procédé selon la revendication 1 dans lequel on prépare un composé choisi dans le groupe constitué par:

(1) Acide α-[1-(4′-chlorobenzoyl)-5-méthoxy-2-méthylindol-3-yl]-N-(morpholinométhyl)-acétohydroxamique

(2) Acide α-[1-(4′-chlorobenzoyl)-5-méthoxy-2-méthylindol-3-yl]-N-(N′,N′-diéthylaminométhyl)acétohydroxamique

(3) Acide α-[1-(4′-chlorobenzoyl)-5-méthoxy-2-méthylindol-3-yl]-N-[(4-méthylpipérazin-1-yl)méthyl]-acétohydroxamique

(4) Acide α-[1-(4′-chlorobenzoyl)-5-méthoxy-2-méthylindol-3-yl]-N-(pipéridinométhyl)acétohydroxamique

(5) Acide α-[1-(4′-chlorobenzoyl)-5-méthoxy-2-méthylindol-3-yl]-N-[(pyrrolidin-1-yl)méthyl]acétohydroxamique

(6) Acide α-[1-(4′-chlorobenzoyl)-5-méthoxy-2-méthylindol-3-yl]-N-(N′,N′-dicyclohexylaminométhyl)-acétohydroxamique

(7) Acide α-[1-(4′-chlorobenzoyl)-5-méthoxy-2-méthylindol-3-yl]-N-[(pyrrol-1-yl)méthyl]acétohydroxamique

(8) Acide α-[1-(4′-chlorobenzoyl)-5-méthoxy-2-méthylindol-3-yl]-N-[(indol-1-yl)méthyl]acétohydroxamique

(9) Acide α-[1-(4′-chlorobenzoyl)-5-méthoxy-2-méthylindol-3-yl]-N-[(isoindol-2-yl)méthyl]acétohydroxamique

(10) Acide α-[1-(4′-chlorobenzoyl)-5-méthoxy-2-méthylindol-3-yl]-N-[(purin-7-yl)méthyl]acétohydroxamique

(11) Acide α-[1-(4′-chlorobenzoyl)-5-méthoxy-2-méthylindol-3-yl]-N-(N′,N′-diméthylaminométhyl)-acétohydroxamique

(12) Acide α-[1-(4′-chlorobenzoyl)-5-méthoxy-2-méthylindol-3-yl]-N-(N′,N′-diisopropylaminométhyl)-acétohydroxamique

(13) Acide α-[1-(4′-chlorobenzoyl)-5-méthoxy-2-méthylindol-3-yl]-N-(N′,N′-dipentylaminométhyl)-acétohydroxamique

(14) Acide α-[1-(4′-chlorobenzoyl)-5-méthoxy-2-méthylindol-3-yl]-N-(N′,N′-dioctylaminométhyl)acétohydroxamique.

11. Procédé selon la revendication 1 dans lequel on prépare un composé choisi dans le groupe constitué par:

(1) Acide α-{*cis*-5-fluoro-2-méthyl-1-[4′-(méthylsulfinyl)benzylidène]indén-3-yl}-N-(pipéridinométhyl)-acétohydroxamique

(2) Acide α-{*cis*-5-fluoro-2-méthyl-1-[4′-(méthylsulfinyl)benzylidène]indén-3-yl}-N-[(N′-benzyl-N′-phényl)aminométhyl]acétohydroxamique

(3) Acide α-{*cis*-5-fluoro-2-méthyl-1-[4′-(méthylsulfinyl)benzylidène]indén-3-yl}-N-(morpholinométhyl)acétohydroxamique

(4) Acide α-{*cis*-5-fluoro-2-méthyl-1-[4′-(méthylsulfinyl)benzylidène]indén-3-yl}-N-[(imidazol-1-yl)-méthyl]acétohydroxamique.

12. Procédé selon la revendication 1 dans lequel on prépare Acide α-(6-méthoxynaphtalén-2-yl)-α-méthyl-N-(N′,N′-diéthylaminométhyl)acétohydroxamique.

13. Procédé selon la revendication 1 dans lequel on prépare un composé choisi dans le groupe constitué par:

(1) Acide α-méthyl-α-(3-phénoxy)phényl-N-(pipéridinométhyl)acétohydroxamique

(2) Acide α-méthyl-α-(3-phénoxy)phényl-N-[(N′-benzyl-N′-phényl)aminométhyl]acétohydroxamique

(3) Acide α-méthyl-α-(3-phénoxy)phényl-N-(morpholinométhyl)acétohydroxamique

(4) Acide α-méthyl-α-(3-phénoxy)phényl-N-[(imidazol-1-yl)méthyl]acétohydroxamique

(5) Acide α-méthyl-α-[4-(2′-méthylpropyl)]phényl-N-(morpholinométhyl)acétohydroxamique

(6) Acide α-méthyl-α-[4-(2′-méthylpropyl)]phényl-N-(pipéridinométhyl)acétohydroxamique

(7) Acide α-[1-méthyl-5-(p-toluoyl)pyrrol-2-yl]-N-(N′,N′-diéthylaminométhyl)acétohydroxamique

(8) Acide α-méthyl-α-[4-(1′oxoisoindol-2′-yl)]phényl-N-(pipéridinométhyl)acétohydroxamique

(9) Acide α-méthyl-α-[4-(1′-oxoisoindol-2′-yl)]phényl-N-(morpholinométhyl)acétohydroxamique.